# EUROPÄISCHE PATENTSCHRIFT

(11) **EP 1 204 668 B1**
(45) Veröffentlichungstag und Bekanntmachung des Hinweises auf die Patenterteilung: **02.04.2003**
(21) Anmeldenummer: 00956407.1
(22) Anmeldetag: 08.08.2000
(51) Int. Cl.: C07F 15/02, C07F 15/06, C08F 4/70, C08F 10/00, C07D 213/53, C07D 401/14

(54) **BISIMIDINOVERBINDUNGEN UND IHRE ÜBERGANGSMETALLKOMPLEXE SOWIE DEREN VERWENDUNG ALS KATALYSATOREN**
BISIMIDINO COMPOUNDS AND THE TRANSITIONAL METAL COMPLEXES THEREOF AS WELL AS THE USE THEREOF AS CATALYSTS
COMPOSES A BASE DE BISIMIDINE, LEURS COMPLEXES DE METAUX DE TRANSITION ET LEUR UTILISATION EN TANT QUE CATALYSEURS

(30) Priorität: 20.08.1999 DE 19939415
(43) Veröffentlichungstag der Anmeldung: 15.05.2002
(73) Patentinhaber: BASF AKTIENGESELLSCHAFT, 67056 Ludwigshafen (DE)
(72) Erfinder: KRISTEN, Marc, Olivier, D-67117 Limburgerhof (DE); GONIOUKH, Andrei, D-67373 Dudenhofen (DE); LILGE, Dieter, D-67117 Limburgerhof (DE); LEHMANN, Stephan, D-67067 Ludwigshafen (DE); BILDSTEIN, Benno, A-6020 Innsbruck (AT); AMORT, Christoph, I-39030 Rodeneck (IT); MALAUN, Michael, A-6473 Wenns (AT)
(86) Internationale Anmeldenummer: EP0007657
(87) Internationale Veröffentlichungsnummer: WO01014391

(56) Entgegenhaltungen:
- WO-A-99/12981

## Beschreibung

Die Erfindung betrifft Bisimidin-Verbindungen, ein Verfahren zu deren Her-stellung, Bisimidinato-Komplexe als Katalysatoren, Verfahren zu deren Her-stellung sowie deren Einsatz in der Polymerisation ungesättigter Verbindungen.

Es besteht großes Interesse an der Entwicklung neuartiger Familien von Katalysatoren für die Polymerisation ungesättigter Verbindungen, um eine bessere Kontrolle über die Eigenschaften von Polyolefinen oder weitere neuartige Produkte zu erhalten.

Die Verwendung von Übergangsmetallverbindungen als katalytisch aktive Substanzen zur Polymerisation ungesättigter Verbindungen ist seit langem bekannt. Beispielsweise werden Ziegler-Nattaoder Phillips-Katalysatoren kommerziell zur Synthese von Polyolefinen eingesetzt. Seit neuerer Zeit werden Metallocene als Polymerisationskatalysatoren eingesetzt, die sich durch hohe Aktivitäten auszeichnen. Mit Hilfe der Metallocene sind Polymere mit enger Molekulargewichtsverteilung und Copolymere mit gleichmäßigem Comonomereinbau zugänglich.

Die Metallocenkatalysatoren haben jedoch für den großtechnischen Einsatz Nachteile. Sie sind beispielsweise gegenüber Verunreinigungen in kommerziell erhältlichen Monomeren, im Prozeßgas und den eingesetzten Lösungsmitteln und gegen Hydrolyse sehr empfindlich. Des weiteren ist der Preis für Metallocene mit Zirkon als Zentralmetall sehr hoch.

Seit einiger Zeit ist bekannt, daß neuartige Eisen- und Cobaltkomplexe, die Bisimidin-Liganden tragen, als Katalysatoren in der Polymerisation ungesättigter Verbindungen sehr aktiv sind.

In V. C. Gibson et al., Chem. Commun. 1998, 849-850 und M. Brookhart et al., J. Am. Chem. Soc. 1998, 120, 4049-4050, sind neue Olefin-Polymerisationskataly-satoren auf Basis von Fe(II) und Co(II) offenbart. Diese Katalysatoren tragen 2,6- Bis(imino)pyridyl-Liganden, die an den Iminostickstoffatomen aryl-substituiert sind, und zeigen hohe Aktivitäten in der Polymerisation von Ethylen. Das erhaltene Polyethylen ist im wesentlichen linear und das Molekulargewicht ist stark abhängig von den Substituenten am Arylrest.

In WO 99/12981 sind Bisimidinato-Komplexe, deren Synthese sowie deren Einsatz in der Polymerisation ungesättigter Verbindungen beschrieben. Es sind zahlreiche Komplexe mit einer Reihe unterschiedlicher Reste offenbart.

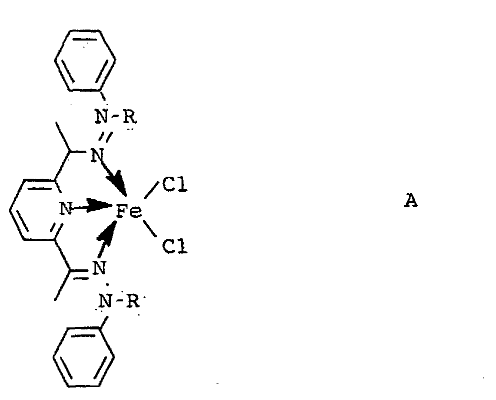

Beispiel 30 und 31 offenbaren Komplexe der Formal A mit R = CH₃ und C₆H₅. Die gezeigten Aktivitäten bei der Polymerisation von Ethylen sind jedoch für technische Anwendungen viel zu gering.

Aufgabe der vorliegenden Erfindung ist es, neue Komplexe mit einem Metall der Gruppen 7, 8, 9 und 10 des Periodensystems der Elemente (spätes Übergangsmetall) als Zentralmetall für die Polymerisation ungesättigter Verbindungen bereitzustellen, die bei der Polymerisation Polymere mit Verzweigungen liefern.

Diese Aufgabe untergliedert sich in die Bereitstellung eines Ligandsystems für diesen Katalysator, sowie ein Verfahren zur Herstellung dieses Ligandsystems und die Bereitstellung eines Verfahrens zur Herstellung des entsprechenden Katalysators.

Diese Aufgabe wird gelöst durch Verbindungen der allgemeinen Formel (I) 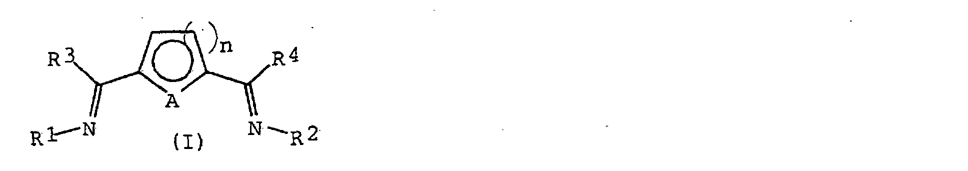 in der die Symbole die folgende Bedeutung haben
- A: Nichtmetall ausgewählt aus N, S, O und P
- R¹: Reste der allgemeinen Formel NR⁵R⁶,
- R²: Reste der allgemeinen Formel NR⁵R⁶ oder NR⁷R⁸, wobei R⁵ bis R⁸ ausgewählt werden aus Alkyl-, Aryl- oder Cycloalkylresten, und wobei
- R⁵ und R⁶: gemeinsam mit dem N-Atom einen 5-, 6- oder 7-gliedrigen Ring bilden, in dem eine oder mehrere der -CH- oder -CH₂-Gruppen durch geeignete Heteroatomgruppen ersetzt sein können, der gesättigt, ungesättigt und unsubstituiert, substituiert oder mit weiteren carbacyclischen oder heterocarbacyclischen 5- oder 6-gliedrigen Ringen, die ihrerseits gesättigt oder ungesättigt und substituiert oder unsubstituiert sein können, anelliert sein kann,
und
- R⁷ und R⁸: unabhängig voneinander Alkyl-, Aryl- oder Cycloalkylreste,
und
- R³, R⁴: unabhängig voneinander H, Alkyl-, Aryl- oder Cycloalkylreste,
und
- n: 1 oder 2.

Die erfindungsgemäßen Bisimidine zeichnen sich dadurch aus, daß sie mindestens eine Stickstoff-Stickstoff-Bindung zwischen mindestens einem der beiden Imin-Stickstoffatome als [=N-NR⁵R⁶] aufweisen, wobei die Substituenten R⁵ und R⁶ gemeinsam einen cyclischen Substituenten bilden.

Diese Verbindungen sind insbesondere als Ligandsysteme zur Herstellung von neuartigen, effizienten Katalysatorsystemen zur Polymerisation oder Copoly-merisation ungesättigter Verbindungen geeignet. Diese neuen Liganden sind leicht herstellbar und ermöglichen eine große Variationsbreite der Reste. Damit ist dieses System sehr variabel und erlaubt das Maßschneidern von Ligandund Komplexsystemen für verschiedene Einsatzzwecke. Mit Hilfe von Verbindungen der allgemeinen Formel (I) als Ligandsystem sind hochaktive Katalysatoren für die Polymerisation ungesättigter Verbindungen zugänglich.

Vorstehend und im Folgenden sind unter Alkylresten im allgemeinen lineare oder verzweigte C₁- bis C₂₀-Alkylreste zu verstehen, bevorzugt C₁- bis C₁₀-Alkylreste, besonders bevorzugt C₁- bis C₈-Alkylreste. Diese Alkylreste können heteroatomsubstituiert sein. Geeignete Alkylreste sind beispielsweise Methyl-, i-Propyl-, t-Butyl-, Trifluormethyl- und Trimethylsilylreste.

Unter Arylresten sind im allgemeinen unsubstituierte und substituierte C₆- bis C₂₀-Arylreste (die Zahl der Kohlenstoffatome bezieht sich dabei auf die Kohlenstoffatome im Arylrest) zu verstehen, bevorzugt C₆- bis C₁₄-Arylreste, die unsubstituiert oder einfach oder mehrfach substituiert sein können, ganz besonders bevorzugt sind mit C₁- bis C₆-Alkylresten sub-stituierte C₆- bis C₁₀-Arylreste wie 4-Methylphenyl, 2,6-Dimethylphenyl, 2,6-Diethylphenyl, 2,6-Diisopropylphenyl, 2-tert.-Butyl-phenyl, 2,6-Di(tert.-butyl)-phenyl oder 2-i-Propyl-6-methylphenyl. Die Arylreste können auch mit Heteroatomen, z.B. mit F substituiert sein.

Unter Cycloalkylresten sind im allgemeinen C₅- bis C₈-Cycloalkylreste zu verstehen (die Zahl der Kohlenstoffatome bezieht sich dabei auf die Kohlenstoffatome im Cycloalkylring), die unsubstituiert oder ein- oder mehrfach mit Alkyl- oder Arylresten oder Heteroatomen substituiert sein können. Bevorzugt sind C₅- bis C₆-Cycloalkylreste.

Gemäß der vorliegenden Erfindung bilden R⁵ und R⁶ gemeinsam mit dem N-Atom einen 5-, 6- oder 7-gliedrigen Ring, in dem eine oder mehrere der -CH- oder -CH₂-Gruppen durch geeignete Heteroatomgruppen ersetzt sein können. Geeignete Heteroatomgruppen sind darin bevorzugt -N- bzw. -NH-Gruppen. Besonders bevorzugt sind keine bis 3 -CH- oder -CH₂-Gruppen durch -N- oder -NH-Gruppen ersetzt.

Der 5-, 6- oder 7-gliedrige Ring kann gesättigt oder ungesättigt sein. Dabei kann der Ring ein- oder mehrfach ungesättigt sein. Bevorzugt sind ungesättigte 5-gliedrige Ringe. Unter ungesättigten Ringen sollen auch, im Falle der 5-gliedrigen Ringe, aromatische Ringe wie unsubstituierte oder substituierte Pyrrolreste und Derivate davon, die besonders bevorzugt sind, verstanden werden.

Der 5-, 6- oder 7-gliedrige Ring kann unsubstituiert, substituiert oder mit weiteren carbacyclischen oder heterocarbacyclischen 5- oder 6-gliedrigen Ringen, die ihrerseits gesättigt oder ungesättigt und substituiert oder unsubstituiert sein können, anelliert sein.

Unter carbacyclischen Ringen sind solche Ringe zu verstehen, die ein reines Kohlenstoffgerüst aufweisen. In den heterocarbacyclischen Ringen sind ein- oder mehrere -CH₂- oder -CH- Gruppen durch Heteroatome, bevorzugt -NH- oder -N-Gruppen, ersetzt. Besonders bevorzugt sind carbacyclischen Ringe oder heterocarbacyclische Ringe mit einem Stickstoffatom im Ringsystem.

Als Substituenten in diesen carbacyclischen und heterocarbacyclischen 5- oder 6-gliedrigen Ringen kommen die vorstehend genannten Alkyl-, Aryl oder Cycloalkylreste in Frage. Die Ringe können einoder mehrfach substituiert sein. Dabei ist 1- bis 3-fache Substitution bevorzugt. Das Ringsystem kann des weiteren ortho- oder ortho- und peri-anelliert sein. Bevorzugt ist das System orthoanelliert, wobei besonders bevorzugt 1 bis 2 Phenylreste an den zentralen 5- oder 6-Ring anelliert sind, wie Indol, Carbazol und Derivate davon.

In einer besonders bevorzugten Ausführungsform ist der durch die allgemeine Formel NR⁵R⁶ beschriebene Ring 5-gliedrig. Ganz besonders bevorzugt ist ein nicht anellierter 5-Ring, insbesondere ein Pyrrolrest oder ein von Pyrrol abgeleiteter Rest, worin keine, eine oder mehrere, bevorzugt 0 bis 3, besonders bevorzugt 0 oder 2 -CH-Gruppen im Pyrrolring durch Stickstoff ausgetauscht sein können. Beispiele dafür sind das Pyrrol- und das Triazol-System. Besonders bevorzugt sind Pyrrolreste oder von Pyrrol abgeleitete Reste, die in der 2- und 5-Position substituiert sind mit: C₁- bis C₆-Alkylgruppen, die linear, verzweigt und mit Heteroatomen - substituiert sein können, elektronenziehende Reste wie Halogen, Nitro, Sulfonat oder Trihalogenmethyl substituiert sind.

Unter den Sulfonatresten kommen insbesondere SO₃R*, SO₃Si(R*)₃, und SO₃⁻ (HN(R*)₃)⁺ in Frage. Besonders geeignet unter diesen sind jeweils SO₃Me, SO₃SiMe₃ und SO₃-(HNEt₃)+. Unter den Trihalogenmethylresten sind Trifluor, Trichlor und Tribrommethyl, insbesondere Trifluormethyl besonders geeignet. Besonders geeignete ortho-Substituenten sind Halogenreste wie der Fluor-, Chlor-, Brom- oder Iodrest. Bevorzugt werden Chlor- oder Bromreste als ortho-Substituenten eingesetzt. Des weiteren sind die jeweiligen ortho-Positionen bevorzugt mit identischen Resten besetzt. Arylgruppen, die unsubstituiert oder ihrerseits mit C₁- bis C₆-Alkylgruppen, die heteroatomsubstituiert sein können, substituiert sind. Bevorzugte Substituenten in der 2- und 5-Position des Pyrrolrings oder eines Derivates davon, bevorzugt Triazol, sind Methyl-, i-Propyl-, t-Butyl-, Phenyl-, substituierte Arylreste, wie sie vorstehend definiert sind.

Gemäß der vorliegenden Erfindung können R³ und R⁴ in der allgemeinen Formel (I) unabhängig voneinander H, Alkyl-, Aryl- oder Cycloalkylreste sein, wobei bevorzugte Reste vorstehend definiert sind. Ganz besonders bevorzugt sind R³ und R⁴ unabhängig voneinander H oder CH₃.

Gemäß der vorliegenden Erfindung kann R* H, Alkyl, Aryl oder Gebalkyl sein, wobei bevorzugte Reste vorstehend definiert sind. Ganz besonders bevorzugt ist R*, CH₃ oder H.

Bevorzugt werden Verbindungen der allgemeinen Formel (I) eingesetzt, in denen A = N oder S ist. Besonders bevorzugt ist A = N. Der zentrale Ring ist bevorzugt ein 6-gliedriger Ring, d.h. n ist bevorzugt 2. Somit sind Pyridinbisimidin-Systeme ganz besonders bevorzugt.

Besonders bevorzugt sind Verbindungen der allgemeinen Formel (Ia) bis (Id) : 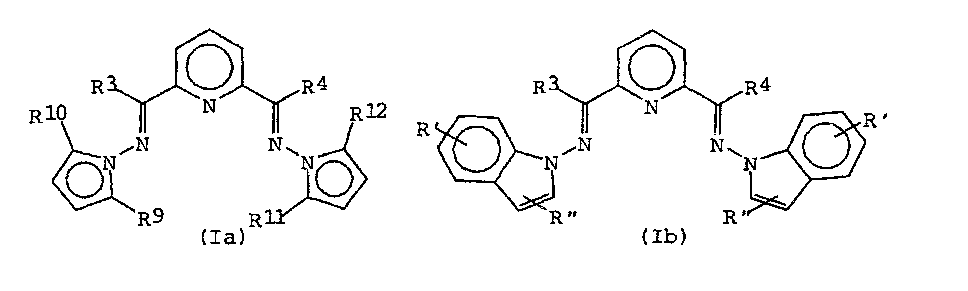 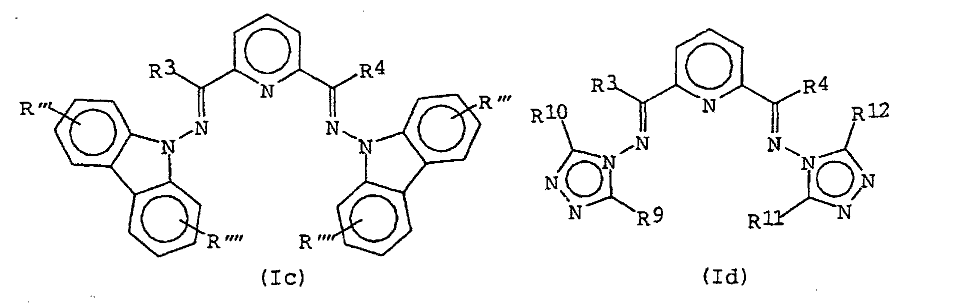 worin R³, R⁴, R⁹ und R¹⁰ unabhängig voneinander C₁- bis C₂₀-Alkylreste, die linear oder verzweigt sein können, bevorzugt C₁- bis C₁₀-Alkylreste, besonders bevorzugt C₁-bis C₈-Alkylreste bedeuten. Diese Alkylreste können heteroatomsubstituiert sind. Geeignete Alkylreste sind beispielsweise Methyl-, i-Propyl-, t-Butyl-, Trifluormethyl- und Trimethylsilylreste. Die Reste R', R'', R''' und R'''' sind H, Alkyl-, Aryl- oder Cycloalkylreste, wie vorstehend definiert.

Die erfindungsgemäßen Verbindungen der allgemeinen Formel (I) 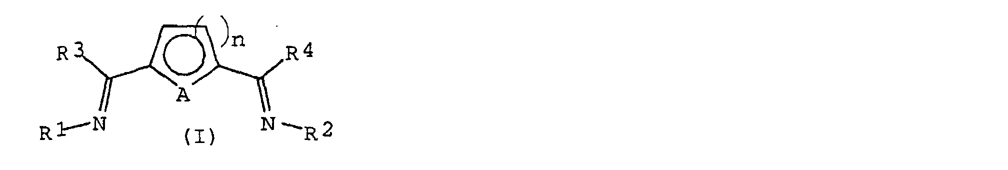 worin
- R¹: Reste der allgemeinen Formel NR⁵R⁶,
- R2: Reste der allgemeinen Formel NR⁵R⁶, NR⁷R⁸ oder Alkyl-, Aryl- oder Cycloalkylreste,
bedeuten und die anderen Symbole die vorstehenden Bedeutungen haben, werden im allgemeinen durch Kondensation der entsprechenden Aminoverbindungen mit den entsprechenden Diketoverbindungen, wie 2,5-Diformylthiophen oder 2,6-Diacetylpyridin, hergestellt. Sie sind synthetisch sehr gut zugänglich und es ist möglich, eine große Zahl verschiedener Verbindungen der allgemeinen Formel (I) in guten Ausbeuten zu synthetisieren.

Dabei ist das bevorzugte Herstellverfahren abhängig von der gewünschten Verbindung der allgemeinen Formel (I). Im Folgenden sind bevorzugte Ausführungsformen zur Herstellung von symmetrischen Verbindungen der allgemeinen Formel I, worin R¹ = R² = NR⁵R⁶ ist, und unsymmetrischen Verbindungen der allgemeinen Formel I, worin R¹ ≠ R² ist und R² ein Rest der allgemeinen Formel NR⁵R⁶, der sich von dem in R¹ unterscheidet, ein Rest der allgemeinen Formel NR⁷R⁸ oder ein Alkyl-, Aryl- oder Cycloalkylrest ist, beschrieben.

In einer bevorzugten Ausführungsform werden symmetrische Verbindungen der allgemeinen Formel (I), worin R¹ = R² ist, durch Umsetzung von Verbindungen der allgemeinen Formel (II)

H₂N―NR⁵R⁶ (II)

worin
R⁵ und R⁶ die vorstehenden Bedeutungen haben,
mit Diketoverbindungen der allgemeinen Formel (III) hergestellt, 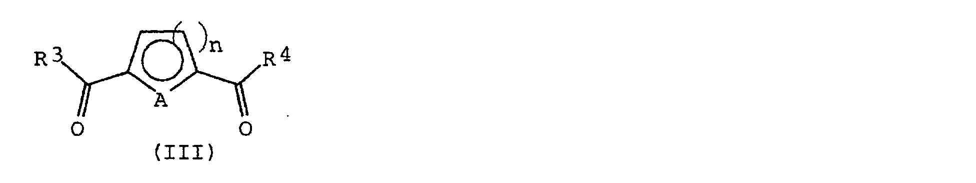 worin
- R³, R⁴: unabhängig voneinander H, Alkyl-, Aryl- oder Cycloaklylreste sind, und
- A: S, N, O oder P ist, und
- n: 1 oder 2 ist.

Das Verfahren ist einstufig und wird unter sauren Reaktionsbedingungen durchgeführt, bevorzugt unter Zugabe einer Mineralsäure oder einer organischen Säure, besonders bevorzugt Ameisensäure, in alkoholischen Lösungsmitteln, bevorzugt in Methanol. Alternativ kann das Verfahren unter Aluminiumtrialkyl-Katalyse durchgeführt werden, wobei bevorzugt Trimethylaluminium eingesetzt wird, in einem aprotischen Lösungsmittel, bevorzugt in Toluol. Das Verhältnis der Verbindung der allgemeinen Formel (II) zu der Verbindung der allgemeinen Formel (III) beträgt 2 : 0,7 bis 1,3, bevorzugt 2 : 0,9 bis 1,1, besonders bevorzugt 2 : 1. Dabei ist die Umsetzung unter sauren Bedingungen in Methanol/Ameisensäure im allgemeinen bevorzugt.

Im allgemeinen wird die Kondensation bei Temperaturen von 0 bis 100°C durchgeführt, bevorzugt von 15 bis 80°C, besonders bevorzugt von 20 bis 40°C. Die Reaktionsdauer beträgt im allgemeinen 20 Min. bis 48 h, bevorzugt 1 h bis 16 h, besonders bevorzugt 2 h bis 14 h. Die genauen Reaktionsbedingungen sind von den jeweils eingesetzten Verbindungen abhängig. Bei Verbindungen der allgemeinen Formeln (II) und (III), die nur langsam zu den gewünschten Verbindungen der allgemeinen Formel (I) kondensieren, ist gegebenenfalls eine Umsetzung unter Aluminiumtrialkyl-Katalyse in aprotischen Lösungsmitteln bevorzugt.

In einer weiteren bevorzugten Ausführungsform werden die unsymmetrischen 1,2-Diimine der allgemeinen Formel (I), worin R¹ ≠ R² ist, in einem zweistufigen Verfahren hergestellt, in dem:
a) in einer ersten Stufe Verbindungen der allgemeinen Formel (II)

   H₂N―NR⁵R⁶ (II)

   worin
   R⁵ und R⁶ die vorstehenden Bedeutungen haben,
   mit Diketoverbindungen der allgemeinen Formel (III)
   mit Diketoverbindungen der allgemeinen Formel (III) hergestellt, 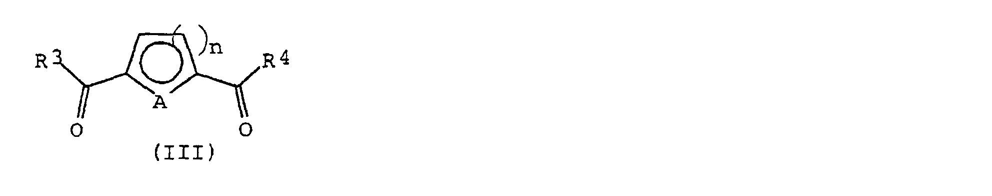 worin
   - R³, R⁴: unabhängig voneinander H, Alkyl-, Aryl- oder Cycloaklylreste sind, und
   - A: S, N, O oder P ist, und
   - n: 1 ode 2 ist,
   in einem Verhältnis der Verbindungen der allgemeinen Formel (II) zu den Verbindungen der allgemeinen Formel (III) von 1 : 0,8 bis 1,2, bevorzugt von 1 : 0,9 : 1,1, besonders bevorzugt von 1 : 1, unter sauren Bedingungen, bevorzugt unter Zusatz von Mineralsäuren oder organischen Säuren, besonders bevorzugt Ameisensäure, in alkoholischer Lösung, bevorzugt in Methanol, zu dem entsprechenden Monoimin umgesetzt werden und das Lösungsmittel anschließend im Vakuum entfernt wird,
   und
b) das Monoimin in einer zweiten Stufe mit Verbindungen der allgemeinen Formel (II), die sich von den in Stufe a) eingesetzten Verbindungen der allgemeinen Formel (II) unterscheiden, oder
   mit Verbindungen der allgemeinen Formel (IV)

   H₂N-NR⁷R⁸ (IV)

   worin
   - R⁷ und R⁸: unabhängig voneinander Alkyl-, Aryl- oder Cycloalkylreste sind, oder
   mit Aminen der allgemeinen Formel (V)

   ¹³-NH₂ (V)

   worin
   - ¹³: ein Alkyl-, ein Aryl- oder ein Cycloalkylrest, wie vorstehend definiert, ist,
in aprotischer Lösung, bevorzugt in Toluol, unter Aluminiumtrialkyl-Katalyse, bevorzugt mit Aluminiumtrimethyl als Katalysator, in einem Verhältnis des Monoimins zu einer Verbindung der allgemeinen Formel (II), (IV) oder der allgemeinen Formel (V) von 1 : 0,8 bis 1,2, bevorzugt von 1 : 0,9 bis 1,1, besonders bevorzugt von 1 : 1 umgesetzt wird.

Im allgemeinen wird die Kondensation in Stufe a) bei Temperaturen von 0 bis 100°C durchgeführt, bevorzugt von 15 bis 80°C, besonders bevorzugt von 20 bis 40°C. Die Reaktionsdauer beträgt im allgemeinen 20 Min. bis 48 h, bevorzugt 1 h bis 16 h, besonders bevorzugt 2 h bis 14 h. Die genauen Reaktionsbedingungen sind von den jeweils eingesetzten Verbindungen abhängig. Die Stufe b) wird im allgemeinen bei Temperaturen von 0 bis 100°C durchgeführt, bevorzugt von 20 bis 80°C, besonders bevorzugt von 30 bis 60°C. Die Reaktionsdauer beträgt im allgemeinen 20 Min. bis 48 h, bevorzugt 1 h bis 8 h, besonders bevorzugt 2 h bis 7 h. Die genauen Reaktionsbedingungen sind wiederum von den jeweils eingesetzten Verbindungen abhängig.

Besonders bevorzugt werden als Verbindungen der allgemeinen Formel (II)

H₂N-NR⁵R⁶ (II)

worin
R⁵ und R⁶ die vorstehenden Bedeutungen haben,

Verbindungen eingesetzt, in denen die Gruppe NR⁵R⁶ ein Pyrrolrest oder ein von Pyrrol abgeleiteter Rest ist, der ganz besonders bevorzugt in der 2- und 5-Position mit C₁- bis C₆-Alkylgruppen, die linear, verzweigt und mit Heteroatomen substituiert sein können, und/oder Arylgruppen, die unsubstituiert oder ihrerseits mit C₁bis C₆-Alkylgruppen, die heteroatomsubstituiert sein können, substituiert ist. Bevorzugte Substituenten in der 2- und 5-Position des Pyrrolrings sind Methyl-, i-Propyl-, t-Butyl-, Phenyl-, substituierte Arylreste, wie sie vorstehend definiert sind.

Solche N-Amino-Pyrrole können beispielsweise durch das folgende zweistufige Verfahren erhalten werden:
i) Umsetzung eines geeigneten 1,4-Diketons mit einer äquivalenten Menge Acetylhydrazin oder Benzoyloxycarbonylhydrazin in Anwesenheit einer katalytischen Menge Säure, bevorzugt p-Toluolsulfonsäure, in einem inerten organischen Lösungsmittel, bevorzugt Toluol, zu dem entsprechenden Acetyl- oder Benzyloxycarbonyl-geschützten N-Amino-Pyrrol;
ii) Hydrolyse des geschützten N-Amino-Pyrrols mit einem Überschuß Base, bevorzugt mit Kaliumhydroxid, in einem hochsiedenden inerten organischen Lösungsmittel, bevorzugt in Ethylenglycol, unter Rückfluß zu dem entsprechenden freien N-Amino-Pyrrol.

Die anschließende Aufarbeitung erfolgt in üblicher Weise.

Als Diketoverbindungen werden in dem erfindungsgemäßen Verfahren Verbindungen der allgemeinen Formel (III) eingesetzt: 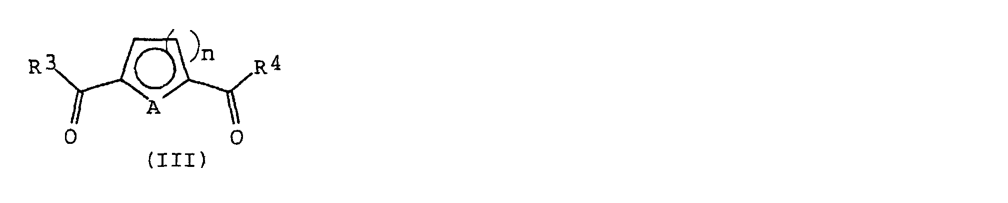 worin
- R³, R⁴: unabhängig voneinander H, Alkyl-, Aryl- oder Cycloaklylreste sind, wobei bevorzugte Reste vorstehend definiert sind; ganz besonders bevorzugt sind R³ und R⁴ H oder CH₃; und
- A: S, N, O oder P, bevorzugt N oder S, besonders bevorzugt N ist, und
- n: 1 oder 2, bevorzugt 2 ist.

Der zentrale Grundkörper der Verbindungen der allgemeinen Formel (III) ist somit bevorzugt ein Pyridingrundkörper, der in 2- und 6-Position substituiert ist.

Die erfindungsgemäßen Verbindungen sind als Liganden für Katalysatoren, die zur Polymerisation ungesättigter Verbindungen eingesetzt werden können, geeignet. Insbesondere sind die erfindungsgemäßen Verbindungen als Liganden für Katalysatoren mit einem Metall der späten Übergangsmetalle, z.B. mit einem Metall der Gruppe 7, 8, 9 und 10 des Periodensystems der Elemente geeignet. Ein weiterer Gegenstand der vorliegenden Erfindung sind daher Verbindungen der allgemeinen Formel (VI), 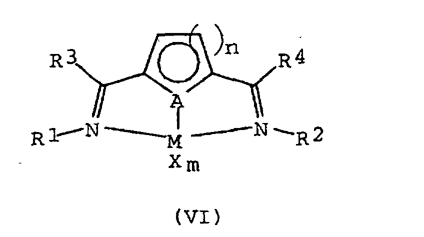 worin die Symbole die folgende Bedeutung haben
- A: Nichtmetall ausgewählt aus N, S, O und P
- R¹: Reste der allgemeinen Formel NR⁵R⁶,
- R2: Reste der allgemeinen Formel NR⁵R⁶, NR⁷R⁸ oder Alkyl-, Aryl- oder Cycloalkylreste,
- R⁵ und R⁶: bilden gemeinsam mit dem N-Atom einen 5-, 6- oder 7-gliedrigen Ring, in dem eine oder mehrere der -CH- oder -CH₂-Gruppen durch geeignete Heteroatomgruppen ersetzt sein können, der gesättigt, ungesättigt und unsubstituiert, substituiert oder mit weiteren carbacyclischen oder heterocarbacyclischen 5- oder 6-gliedrigen Ringen, die ihrerseits gesättigt oder ungesättigt und substituiert oder unsubstituiert sein können, anelliert sein kann, und
- R⁷, R⁸: unabhängig voneinander Alkyl-, Aryl- oder Cycloalkylreste,
und
- R³, R⁴: unabhängig voneinander H, Alkyl-, Aryl- oder Cycloalkkylreste,
- n: 1 oder 2;
- M: Übergangsmetall der Gruppe 7, 8, 9, 10 des Perioden-systems der Elemente,
und
- x: ein Halogenid oder ein C₁- bis C₆-Alkylrest,
- m: Wertigkeit des Metalls, bevorzugt 2 oder 3.

Das Übergangsmetall M der Gruppe 7, 8, 9 oder 10 des Periodensystems der Elemente ist bevorzugt Ru, Mn, Co, Fe, Ni oder Pd. Dabei können die Metalle in den folgenden Wertigkeiten eingesetzt werden: Fe(II), Fe(III), Co(I), Co(II), Co(III), Ru(II), Ru(III), Ru(IV), Mn(I), Mn(II), Mn(III), Mn(IV), Ni(II), Pd(II). Besonders bevorzugt sind Fe und Co und m = 2. Die Liganden X können unabhängig voneinander Halogenid oder Alkylreste sein. Bevorzugt handelt es sich um Chlorid-, Bromid- oder Methylreste. Besonders bevorzugt ist als Gruppe MXₘ: MnCl₂, FeCl₃, CoCl₃, PdCl₂, NiCl₂ oder CoCl₂, FeCl₂.

Bevorzugte Reste R¹, R², R³ und R⁴ haben die vorstehend genannten Bedeutungen.

Ganz besonders bevorzugt sind Verbindungen der allgemeinen Formeln (VIa) bis (VId) : 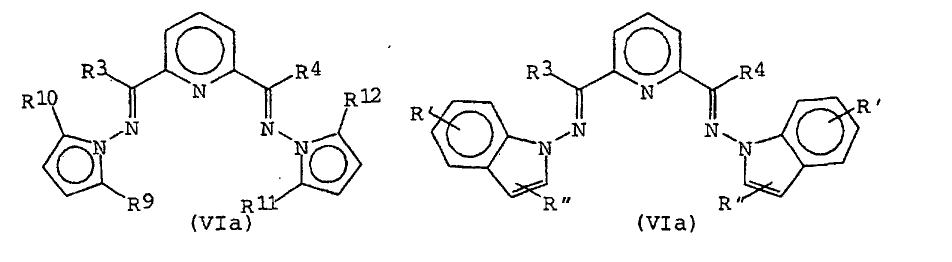 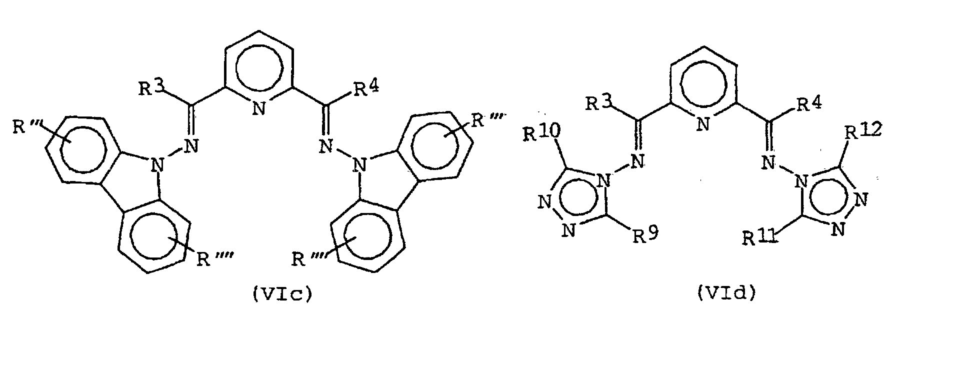 worin
- R³, R⁴: unabhängig voneinander H, Alkyl- oder Arylreste, wobei bevorzugte Reste vorstehend definiert sind,
und
- R⁹, R¹⁰, R¹¹ und R¹²: unabhängig voneinander C₁- bis C₆-Alkylreste bedeuten, wobei bevorzugte Reste vorstehend definiert sind, und
- R', R'', R''', R'''' H,: Alkyl-, Aryl- oder Cycloalkylreste, wobei bevorzugte Reste vorstehend definiert sind, bedeuten,
und
- MX²: MnCl₂, CoCl₂ oder FeCl₂, besonders bevorzugt FeCl₂ und CoCl₂ ist.

Diese Komplexe sind nach Aktivierung mit einem Aktivator (Cokatalysator) hochaktiv in der Polymerisation ungesättigter Verbindungen. Dabei zeigt sich eine starke Abhängigkeit der erhaltenen Polymere von der Struktur des Liganden. Somit sind durch geringe Variationen des Liganden eine große Zahl katalytisch aktiver Verbindungen zugänglich, die die Herstellung von Polymeren und Oligomeren mit einem breiten Spektrum verschiedener Eigenschaften ermöglichen.

Die Herstellung der erfindungsgemäßen Verbindungen der allgemeinen Formel (VI) erfolgt üblicherweise durch Umsetzung der entsprechenden Verbindungen der allgemeinen Formel (I) mit Übergangsmetallsalzen von Metallen der Gruppen 7, 8, 9 und 10 des Periodensystems der Elemente.

In einer bevorzugten Ausführungsform wird eine als Ligand geeignete Verbindung der allgemeinen Formel (I) in einem organischen Lösungsmittel, z.B. Tetrahydrofuran (THF) oder Methylenchlorid, mit einem entsprechenden Metallsalz, z.B. MnCl₂, FeCl₃, CoCl₃, CoCl₂, NiCl₂, PdCl₂, FeCl₂, FeCl₂-THF-Komplex zusammengegeben. Das Molverhältnis von Ligand zu Metallsalz beträgt im allgemeinen 1,5:1 bis 1:1,5, bevorzugt 1,2:1 bis 1:1,2, besonders bevorzugt ca. 1:1. Die Reaktionsmischung wird im allgemeinen bei Temperaturen von Raumtemperatur bis 50°C, bevorzugt von Raumtemperatur bis 40°C, besonders bevorzugt bei Raumtemperatur für im allgemeinen 0,5 Stunden bis 16 Stunden, bevorzugt 1 bis 6 Stunden, besonders bevorzugt 1 bis 3 Stunden gerührt. Die Aufarbeitung erfolgt in üblicher Weise, z.B. durch Entfernung des Lö-sungsmittels im Vakuum, Waschen des Rückstandes mit einem Lösungsmittel, in dem der Rückstand (Produkt) weitgehend unlöslich ist, z.B. mit Diethylether, gegebenenfalls Digerieren in einem unpolaren Lösungsmittel, z.B. Hexan, Abfiltrieren, Waschen und Trocknen.

Die erfindungsgemäßen Metallkomplexe der allgemeinen Formel (VI) sind leicht zugänglich und als Katalysatoren für die Polymerisation ungesättigter Verbindungen geeignet. Sie zeichnen sich bei der Polymerisation bzw. Copolymerisation ungesättigter Verbindungen durch eine überraschend hohe Produktivität aus. Des weiteren zeichnen sich eine Reihe der erfindungsgemäßen Komplexe bei der Copolymerisation durch eine hohe mit ihnen erzielbare Copolymereinbaurate aus. Schon leichte Variationen am Ligandgerüst des Metallkomplexes eröffnen die Herstellung einer breiten Palette von Polymeren mit unterschiedlichen Eigenschaften, so daß es möglich ist, einen Katalysator für ein Polymer mit den gewünschten Eigenschaften "maßzuschneidern".

Wählt man beispielsweise Eisenkomplexe mit 2,5-Di-iso-Propyl-Pyrrol-Liganden, so erhält man bei der Polymerisation von Ethylen Polymere mit einer relativ hohen Molmasse im Bereich von M_{w} = ca. 10⁵. 2,5-Dimethyl-Pyrrol-Komplexe des Eisens liefern bei der Polymerisation von Ethylen bei Raumtemperatur und Normaldruck Oligomere mit einem M_{w} von 3000 bis 3500 g/mol (bestimmt nach Gelpermeationschromatographie (GPC)) und einer für single-site-Katalysatoren engen Molmassenverteilung von Q = 2 bis 5, bevorzugt von Q = 2 bis 3. Diese Oligomere zeichnen sich besonders durch ihre ungewöhnliche Struktur aus. Sie zeigen ein besonders hohes Maß an Verzweigungen, die auch bei der Homopolymerisation von Ethylen zu beobachten sind. Besonders auffällig ist der sehr hohe Anteil an Verzweigungen, die länger als 6 Kohlenstoffatome sind. Bei anderen (als den erfindungsgemäßen) Katalysatorsystemen, die Polyolefine mit einem hohen Verzweigungsgrad liefern (wie Ni- und Pd-Diimin-Systeme), sind die Methylverzweigungen mit Abstand am häufigsten und die längeren Verzweigungen treten nur in geringem Maß auf. Außerdem weisen die erfindungsgemäßen Oligomere zusätzlich eine große Zahl von ungesättigten Endgruppen auf, die den weiteren Einsatz als Monomer in Polymerisationen oder auch eine chemische Funktionalisierung ermöglichen.

Durch Variation des Liganden, z.B. Eisen-Komplex mit einem Carbazol-Substituenten, können Polymere erhalten werden, die kürzerkettige Oligomere (Flüssigkeiten) liefern.

Andere Katalysatorsysteme, z.B. Co-Komplexe mit Carbazol-Gruppen, liefern sehr kurzkettige Oligomere in einem Bereich von im allgemeinen 6 bis 18 Kohlenstoffatomen, vorzugsweise mit einem Maximum an 8 Kohlenstoffatomen.

Bei der Copolymerisation mit a-Olefinen findet man z.B. bei einem Eisen-Isopropylmethylpyrrol-System, einen hervorragenden Einbau von beispielsweise Hexen.

Durch einfache Variation des Ligandgerüstes stehen also Katalysatoren zur Herstellung unterschiedlichster Polymere zur Verfügung. Diese Katalysatoren zeichnen sich darüberhinaus durch eine sehr hohe Aktivität aus, die in vielen Fällen die von vergleichbaren Systemen übertrifft. Außerdem wurden Cobalt-Komplexe mit einer extrem hohen Aktivität gefunden. Bei literaturbekannten Verbindungen ist die Aktivität der Co-Komplexe üblicherweise um mindestens eine Zehnerpotenz schlechter als die von analogen Fe-Komplexen (V. C. Gibson et al., Chem. Commun. 1998, 849-850 und M. Brookhart et al., J. Am. Chem. Soc. 1998, 120, 4049-4050).

Ein weiterer Gegenstand der vorliegenden Erfindung ist daher die Verwendung von Verbindungen der allgemeinen Formel (VI) als Katalysatoren in einem Verfahren zur Polymerisation ungesättigter Verbindungen, sowie ein Verfahren zur Herstellung von Polyolefinen durch Polymerisation ungesättigter Verbindungen in Anwesenheit des erfindungsgemäßen Katalysators und eines Aktivators.

Als Aktivatoren (Cokatalysatoren) sind insbesondere starke, neutrale Lewis-säuren, ionische Verbindungen mit lewissauren Kationen und ionische Ver-bindungen mit Brönsted-Säuren als Kationen geeignet.

Als starke, neutrale Lewissäuren sind Verbindungen der allgemeinen Formel (VII) bevorzugt,

M'X¹X²X³ (VII)

in der die Symbole die folgende Bedeutung haben
- M': ein Element der III. Hauptgruppe des Periodensystems der Elemente, bevorzugt B, Al oder Ga, besonders bevorzugt B,
- X¹, X², X³: unabhängig voneinander Wasserstoff, C₁- bis C₁₀-Alkyl, C₆- bis C₁₅-Aryl, Alkylaryl, Arylalkyl, Halogenalkyl oder Halogenaryl mit jeweils 1 bis 10 Kohlenstoffatomen im Alkylrest und 6 bis 20 Kohlenstoffatomen im Arylrest oder Fluorid, Chlorid, Bromid oder Iodid, bevorzugt sind Halogenaryle, besonders bevorzugt ist Pentafluorphenyl.

Ganz besonders bevorzugt sind Verbindungen der allgemeinen Formel (VII), in der X¹, X², X³ gleich sind, bevorzugt Tris(pentafluorphenyl)boran.

Eine weitere bevorzugt als Aktivator (Cokatalysator) eingesetzte neutrale Lewissäure ist "R¹⁴AlO" (Alkylaluminoxan), worin R¹⁴ ein C₁- bis C₂₅-Alkyl-, bevorzugt ein C₁- bis C4-Alkylrest, besonders bevorzugt ein Methylrest (Methylaluminoxan) ist.

Geeignete ionische Verbindungen mit lewissauren Kationen sind Verbindungen der allgemeinen Formel (VIII),
in der die Symbole die folgenden Bedeutungen haben
- Y: ein Element der I. bis VI. Hauptgruppe oder der I. bis VIII. Nebengruppe des Periodensystems der Elemente,

Q₁ bis Q_{z} einfach negativ geladene Reste wie C₁- bis C₂₈-Alkyl, C₆- bis C₁₅-Aryl, Alkylaryl, Arylalkyl, Halogenalkyl, Halogenaryl mit jeweils 6 bis 20 Kohlenstoffatomen im Aryl- und 1 bis 28 Kohlenstoffatomen im Alkylrest, C₁- bis C₁₀-Cycloalkyl, welches gegebenenfalls mit C₁- bis C₁₀-Alkylgruppen substituiert sein kann, Halogenid, C₁- bis C₂₈-Alkoxy, C₆- bis C₁₅-Aryloxy, Silyl- oder Mercaptylgruppen,
- a: ganze Zahlen von 1 bis 6,
- z: ganze Zahlen von 0 bis 5,
- d: Differenz von a-z, wobei d jedoch größer oder gleich 1 ist,

Besonders geeignet sind Carboniumkationen, Oxoniumkationen und Sulfoniumkationen sowie kationische Übergangsmetallkomplexe. Insbesondere sind das Triphenylmethylkation, das Silberkation und das 1,1'-Dimethyl-ferrocenylkation zu nennen. Bevorzugt besitzen sie nicht koordinierende Gegenionen, insbesondere Borverbindungen, wie sie auch in WO 91/09882 genannt werden, bevorzugt Tetrakis(pentafluorophenyl)borat.

Ionische Verbindungen mit Brönsted-Säuren als Kation und vorzugsweise ebenfalls nicht koordinierenden Gegenionen sind ebenfalls in WO 91/09882 genannt, bevorzugtes Kation ist N,N-Dimethylanilinium.

Die Menge an Aktivator beträgt bevorzugt 0,1 bis 10 Äquivalente, besonders bevorzugt 1 bis 2 Äquivalente, für Borate, bezogen auf den Katalysator (VI). Für Alkylaluminoxane, insbesondere Methylaluminoxan, beträgt die Menge an Aktivator im allgemeinen 50 bis 1000 Äquivalente, bevorzugt 100 bis 500 Äquivalente, besonders bevorzugt 100 bis 300 Äquivalente, bezogen auf den Katalysator (VI).

Das erfindungsgemäße Polymerisationsverfahren ist zur Herstellung von Homo- oder Copolymeren geeignet. Bevorzugt eingesetzte ungesättigte Verbindungen bzw. Kombinationen ungesättigter Verbindungen sind dabei ungesättigte Ver-bindungen ausgewählt aus Ethylen, C₃- bis C₂₀-Monoolefinen, Ethylen und C₃- bis C₂₀-Monoolefinen, Cycloolefinen, Cycloolefinen und Ethylen und Cyclo-olefinen und Propylen. Bevorzugte C₃- bis C₂₀-Monoolefine sind Propylen, Buten, Hexen und Octen und bevorzugte Cycloolefine sind Norbonen, Norbonadien und Cyclopenten.

Die vorstehend genannten Monomere können mit Monomeren, die eine Carbonylgruppe aufweisen, wie Estern, Carbonsäuren, Kohlenmonoxid und Vinylketonen copolymerisiert werden. Dabei sind die folgenden Kombinationen von ungesättigten Verbindungen bevorzugt: Ethylen und ein Alkylacrylat, insbesondere Methylacrylat, Ethylen und eine Acrylsäure, Ethylen und Kohlen-monoxid, Ethylen, Kohlenmonoxid und ein Acrylatester oder eine Acrylsäure, insbesondere Methylacrylat sowie Propylen und Alkylacrylat, insbesondere Methylacrylat. Des weiteren sind Acrylnitril und Styrol als Comonomere geeignet.

Je nach Reaktionsbedingungen und eingesetzten Monomeren ist es möglich, mit dem erfindungsgemäßen Verfahren Homopolymere, statistische Copolymere oder Blockcopolymere zu erhalten.

Die Polymerisation wird unter allgemein üblichen Bedingungen in Lösung, z.B. als Hochdruckpolymerisation im Hochdruckreaktor oder Hochdruckautoklav, inSuspension oder in der Gasphase (z.B. GPWS-Polymerisationsverfahren) durchgeführt. Die entsprechenden Polymerisationsverfahren können als Batch-Verfahren, halbkontinuierlich oder kontinuierlich durchgeführt werden, wobei die Vorgehensweisen aus dem Stand der Technik bekannt sind.

Die erfindungsgemäßen Katalysatorsysteme können in Form von Vollkatalysatoren oder Trägerkatalysatoren, in Abhängigkeit von den Polymerisations-bedingungen, eingesetzt werden.

Als Trägermaterialien werden bevorzugt feinteilige Feststoffe eingesetzt, deren Teilchendurchmesser im Bereich von im allgemeinen 1 bis 200 mm liegen, bevorzugt von 30 bis 70 mm.

Geeignete Trägermaterialien sind beispielsweise Kieselgele, bevorzugt solche der Formel SiO₂ · a Al₂O₃, worin a für eine Zahl im Bereich von 0 bis 2 steht, bevorzugt von 0 bis 0,5; es handelt sich also um Alumosilikate oder Siliciumdioxid. Derartige Produkte sind im Handel erhältlich, beispielsweise Silica Gel 332 von Grace oder ES 70x von Crosfield.

Diese Trägermaterialien können zur Entfernung von adsorbiertem Wasser einer thermischen oder chemischen Behandlung unterzogen oder calciniert werden, wobei bevorzugt eine thermische Behandlung bei 80 bis 200°C, besonders bevorzugt bei 100 bis 150°C, durchgeführt wird.

Andere anorganische Verbindungen wie Al₂O₃ oder MgCl₂ oder Mischungen, die diese Verbindungen enthalten, können ebenfalls als Trägermaterialien eingesetzt werden.

Die Katalysatoren der allgemeinen Formel (VI) können dabei "in situ" hergestellt und direkt, ohne vorherige Isolierung, in der Polymerisation eingesetzt werden. Die Katalysatoren können auch in Anwesenheit des Trägermaterials "in situ" hergestellt werden.

Als Lösungsmittel sind insbesondere aprotische organische Lösungsmittel geeignet. Dabei können das Katalysatorsystem, die oder das Monomere und das Polymer in diesen Lösungsmitteln löslich oder unlöslich sein, die Lösungsmittel sollten jedoch nicht an der Polymerisation teilnehmen. Geeignete Lösungsmittel sind Alkane, Cycloalkane, ausgewählte halogenierte Kohlenwasserstoffe und aromatische Kohlenwasserstoffe. Bevorzugte Lösungsmittel sind Hexan, Toluol und Benzol, besonders bevorzugt ist Toluol.

Die Polymerisationstemperaturen bei der Lösungspolymerisation liegen im allgemeinen in Bereichen von -20 bis 350°C, bevorzugt von 0 bis 350°C, besonders bevorzugt von +20 bis 180°C, ganz besonders bevorzugt von Raumtemperatur bis 80°C. Der Reaktionsdruck beträgt im allgemeinen 0,1 bis 5000 bar, bevorzugt 0,1 bis 3000 bar, besonders bevorzugt 1 bis 200 bar, ganz besonders bevorzugt 5 bis 40 bar. Die Polymerisation kann in jeder für die Polymerisation ungesättigter Verbindungen geeigneten Apparatur durchgeführt werden.

Zur Kontrolle des Molekulargewichts der Polymere kann die Polymerisation in Anwesenheit von Wasserstoffgas durchgeführt werden, das als Kettentransf er-reagenz wirkt. Üblicherweise ist dabei das mittlere Molekulargewicht umso niedriger, je höher die Wasserstoffkonzentration ist.

Des weiteren können weitere in dem entsprechenden Polymerisationsverfahren übliche Hilfsmittel eingesetzt werden.

Das erfindungsgemäße Polymerisationsverfahren eröffnet einen Zugang zu Polyolefinen mit neuartigen Strukturen und Eigenschaften. Ein weiterer Gegen-stand der vorliegenden Erfindung sind daher Polymere, herstellbar nach dem erfindungsge-mäßen Verfahren.

Die nachfolgenden Beispiele erläutern die Erfindung zusätzlich.

### Beispiele

(Die Numerierung der Beispielverbindungen erfolgt unabhängig von der Numerierung der Verbindungen in der vorstehenden Beschreibung)

### a) Synthese der 1,4-Diketone (Figur 1)

Acetonylaceton (1) ist kommerziell erhältlich (Aldrich),die Ketone, 2-7, wurden nach Literaturvorschriften (T.Saegusa,Y.Ito,T.Konsoke, J.Am. Chem. Soc.,97 (1975) 2912; H. Stetter, M. Schreckenberger, Chem. Ber., 107 (1974) 2453; H.Stetter, H.Kuhlmann, Chem. Ber., 109 (1976) 3426; H. Stetter, Angew. Chem., 21 (1976) 695; H. Stetter, F. Jonas,Chem.Ber., 114 (1981) 564) synthetisiert.

### 1-(2-Methylphenyl)pentan-1,4-dion (5) :

25g (208 mmol) o-Tolylaldehyd, 14,58g (208 mmol) Methylvinylketon, 21,05g (208 mmol) Triethylamin, 5,4g (21,4 mmol) 3-Ethyl-5-(2-hydroxyethyl)-4-methylthiazoliumbromid wurden in der oben angegebenen Reihenfolge in 100 ml DMF gelöst und 12 Stunden bei 80°C gerührt. Anschließend wurde das Reaktionsgemisch mit 400 ml Wasser versetzt und zweimal mit Methylenchlorid extrahiert. Die vereinigten Methylenchloridphasen wurden mit 10%iger Schwefelsäure, gesättigter Natriumhydrogencarbonatlösung gewaschen und über Natrium-sulfat getrocknet. Die Reinigung erfolgte nach dem Abziehen des Lösungsmittels am Rotationsverdampfer durch fraktionierte Destillation.

Ausbeute: 20,52g (107 mmol) 52%, C₁₂H₁₄O₂, Siedepunkt: 93-96°C (Hochvakuum), ¹H NMR (CDCl₃) : δ 2.07 (m 3H CH₃), 2.34 (s 3H CH₃ ), 2.68 (m 2H CH₂), 2.98 (m 2H CH₂), 7.09-7.58 (m 4H Phenyl); M⁺ = 190 m/z.

### b) Allgemeine Arbeitsvorschrift zur Synthese geschützter Pyrrole (Figur 1) (8-11)

5-15g eines 1,4-Diketons (3-7), 10 mg p-Toluolsulfonsäure und 1,5 Äquiv. Acetylhydrazid wurden in 100 ml Toluol 48 Stunden mit einem Wasserabscheider unter Rückfluß erhitzt. Nach dem Erkalten wurde entweder der entstandene Niederschlag aus der Reaktionsmischung abfiltriert oder mit Methylenchlorid extrahiert.

### 1-Acetamido-2-methyl-5-phenylpyrrol (8) :

Reaktionsbedingungen: 48 Stunden Rückfluß, Extraktion mit Methylenchlorid.
Ausbeute: 4,3g (25mmol) 51%, C₁₃H₁₄N₂O, Schmelzpunkt: 159°C, ¹H NMR (DMSO-d₆) : δ 1.27 (s 3H Methyl), 1.37 (s 3H Methyl), 5.19 (d ¹H Pyrrol), 5.47 (d 1H Pyrrol), 6.53-6.80 (m, 5H Phenyl), 10.16 (s 1H N-H); MS: M⁺ = 214 m/z.

### 1-Acetamido-2-methyl-5-isopropylpyrrol (9) :

Reaktionsbedingungen. 48 Stunden Rückfluß, Extraktion mit Methylenchlorid. Ausbeute: 8,11g (45 mmol) 53%; C₁₀H₁₆N₂O, Schmelzpunkt: 81°C, ¹H NMR (DMSO-d₆) : δ 1.06 (d 3H CH(CH₃)₂) (J = 7 Hz), 1.11 (d 3H CH(CH₃)₂) (J = 7 Hz), 1.95 (s 3H CH₃), 2.00 (s 3H CH₃), 2.63 (m 1H CH(CH₃)₂), 5.60 (d 1H Pyrrol), 5.63 (d 1H Pyrrol), 10.13 (s breit 1H N-H); MS: M⁺ = 180 m/z.

### 1-Acetamido-2-methyl-5-(2-methylphenyl)pyrrol (10) :

Reaktionsbedingungen. 37 Stunden Rückfluß, Extraktion mit Methylenchlorid.
Ausbeute: 10,3g (45 mmol) 37%, C₄H16N₂O, Schmelzpunkt: 146°C, ¹H NMR (CDCl₃), δ 1.52, 1.88; 2.15; 2.20; 2.25, 2.26 (s, 9H, CH₃); 5.97, 6.04 (m, 2H, Pyrrol-H); 7.14-7.23 (m, 4H, Phenyl-H); 7.65-7.82 (br. s, 1 H, N-H). Gemäß NMR-Spektrum liegt ein Gemisch zweier Isomeren im Verhältnis 0,8 : 1 vor. MS: M⁺ = 228 m/z.

### 1-Acetamido-2,3,5-triphenylpyrrol (11) :

Reaktionsbedingungen: 48 Stunden Rückfluß, Abfiltrieren des nach dem Erkalten entstan-denen Niederschlages. Ausbeute: 38%, C₂₄H₂₀N₂O, Schmelzpukt: 255°C, ¹H NMR (CDCl₃) : δ 1.25 (s 3H Methyl), 6.01 (s 1H Pyrrol), 6.58-6.95 (m 15H Phenyl), 8.08 (s 1H N-H); MS: M⁺ = 352 m/z.

### c) Allgemeine Arbeitsvorschrift zur Synthese der Aminopyrrole (Figur 1) (12-18)

5-15g Amids (8-11) wurden in Glykol mit 10 Äquiv. Kaliumhydroxid bis zur vollständigen Umsetzung (DC-Kontrolle) Rückfluß erhitzt. Durch Zugabe von 200 ml Wasser und anschließende mehrmalige Extraktion mit Methylenchlorid kann das Produkt analysenrein erhalten werden.

### 1-Amino-2-methyl-5-phenylpyrrol (14) :

Reaktionsbedingungen: 36 Stunden Rückfluß.

Ausbeute: 2,82g (16.3 mmol) 88 %, C₁₁H₁₂N₂, Schmelzpunkt: 92°C, ¹H NMR (DMSO-d₆): δ 2.23 (s 3H Methyl), 5.48 (s 2H NH₂), 5.75 (d 1H Pyrrol), 6.04 (d 1H Pyrrol), 7.05-7.68 (m 5H Phenyl); MS: M⁺ = 172 m/z.

### 1-Amino-2-methyl-5-isopropylpyrrol (15) :

Reaktionsbedingungen: 16 Stunden Rückfluß.

Ausbeute: 5,43g (39.3 mmol) 88%, C₈H₁₄N₂, Schmelzpunkt: 21-24°C, ¹H NMR (CDCl₃): δ 1.24 (d 6H CH(CH₃)₂ J = 7 Hz), 2.23 (s 3H CH₃), 3.10 (Septett 1H CH(CH₃)₂, 4.09 (s breit 2H NH₂), 5.70 (d 1H Pyrrol J = 3Hz), 5.74 (d 1H Pyrrol J = 3Hz); MS: M⁺ = 138 m/z.

### 1-Amino-2-methyl-5-(2-methylphenyl)pyrrol (16):

Reaktionsbedingungen: 12 Stunden Rückfluß

Ausbeute: 7,54g (33 mmol) 37%, C₁₄H₁₆N₂O, Schmelzpunkt:146°C, ¹H NMR (CDCl₃) : (2 Isomere im Verhältnis 0.8:1) δ 1.52 (s 3H CH₃), 1.88 (s 3H CH₃), 2.15 (s 3H CH₃), 2.19 (s 3H CH₃), 2.24 (s 3H CH₃), 2.26 (s 3H CH₃), 5.95-6.06 (m 4H Pyrrol), 7.13-7.23 (m 8H Phenyl), 7.65 (s 1H breit N-H), 7.82 (s 1H breit N-H); MS: M⁺ = 228 m/z.

### 1-Amino-2,3,5-triphenylpyrrol (18) :

Reaktionsbedingungen: 36 Stunden Rückfluß.

Ausbeute: 2,62g (8.4 mmol) 85 %, C₂₂H₁₈N₂, Schmelzpunkt: 179°C, ¹H NMR (CDCl₃) : δ 5.53 (s 2H NH₂), 6.46 (s 1H Pyrrol), 7.05-7.79 (m 15H Phenyl) ; MS: M⁺ = 310 m/z.

### d) Allgemeine Arbeitsvorschrift zur Synthese der 2,6-Diacetylpyridin-bis-(2,5-R-azol-1-ylimine) (Figur 2) (22-31)

2-3 g eines N-Aminoazols (12-21) (2,2 mequ) und ein Moläquiv. Diacetylpyridin wurden in möglichst wenig Methanol (4-8) ml gelöst und mit wenigen Tropfen Ameisensäure für mindestens 12 Stunden gerührt. Je nach Substitution am Pyrrol mußte die Reaktionslösung auch Rückfluß erhitzt werden. Das Diimin konnte in den meisten Fällen nach Ende der Reaktion abfiltriert und am Hochvakuum getrocknet werden. Zur Vervollständigung der Reaktion wurde in einigen Fällen noch N-Aminopyrrol zugegeben, oder das Diimin aus einem geeigeneten Lösungsmittel umkristallisiert, um das Monoimin abzutrennen. Diphenyl- und Triphenylaminopyrrol wurden in Propionsäure bis zu 48 Stunden Rückfluß erhitzt. Reaktions-bedingungen, Ausbeute und spektroskopische Daten werden bei den einzelnen Verbindungen angeführt.

2,6-Diacetylpyridin-bis-(2,5-dimethylpyrrol-1-ylimin) (22) :

### Durchführung wie oben beschrieben:

Reaktionsbedingungen: Raumtemperatur, 16 Stunden; das Diimin wurde abfiltriert, mit kaltem Methanol gewaschen und am Hochvakuum getrocknet ; Ausbeute 77,1 %, Schmelzpunkt: 174-178 °C; C₂₁H₂₅N₅ ; ¹H NMR (CDCl₃) : δ 2.1 (s 12H Methyl), 2.45 (s 6H CH₃C=N), 5.95 (s 4H Pyrrol), 7.95 (t 1H p Pyridin), 8.51 (d 2H m Pyridin); MS: M⁺ = 347 m/z.

### 2,6-Diacetylpyridin-bis-(2,5-diisopropylpyrrol-1-ylimin) (23) :

### Durchführung wie oben beschrieben:

Reaktionsbedingungen: Raumtemperatur 12 Stunden, Rückfluß 2 Stunden; das Diimin wurde bei Raumtemperatur abfiltriert, mit Methanol gewaschen und am Hochvakuum getrocknet; Ausbeute 72,5 %, Schmelzpunkt: 198-203 °C; C₂₉H₄₁N₅; ¹H NMR (CDCl₃): δ 1.20 (d 24H CH(CH₃), 2.32 (s 6H CH₃C=N), 2.64 (m 4H CH(CH₃)), 5.94 (s 4H Pyrrol), 7.95 (t 1H p Pyridin), 8.51 (d 2H m Pyridin); MS: M⁺ = 460 m/z.

### [2,6-Diacetylpyridin-bis-(2-methyl-5-phenylpyrrol-1-ylimin)] (24) :

Zu einer Lösung von 1,5g (8,7 mmol) 1-Amino-2-methyl-5-phenylpyrrol (14) in 30 ml Methanol wurden 0,71g (4,4 mmol) Diacetylpyridin und 3 Tropfen Ameisensäure gegeben. Nach vier Tagen Rückfluß erhitzen wurde das Reaktionsgemisch auf -18°C abgekühlt und der entstandene Niederschlag abfiltriert. Durch Einengen und nochmaliges Abkühlen konnte das Produkt vollständig abgetrennt werden.

Ausbeute: 0,83g (1,8 mmol) 40%, C₃₁H₂₉N₅, Schmelzpunkt: 115°C; ¹H NMR(DMSO-d₆): δ 1.99 (s 6H Methyl), 2.06 (s 6H Methyl), 6.00 (d 2H Pyrrol), 6.31 (d 2H Pyrrol), 7.04-7.38 (m 10H Phenyl) 8.16 (m 2H Pyridin), 8.47 (m 2H Pyridin); MS: M⁺ = 471 m/z.

### [2,6-Diacetylpyridin-bis-(2-methyl-5-isopropylpyrrol-1-ylimin)] (25) :

Zu einer Lösung von 2,71g (19,5 mmol) 1-Amino-2-methyl-5-isopropylpyrrol (15) in 50 ml Methanol wurden 1,59g (9,7 mmol) Diacetylpyridin und 5 Tropfen Ameisensäure gegeben. Nach vier Tagen Rückfluß erhitzen wurde das Reaktionsgemisch auf -18°C abgekühlt und der entstandene Niederschlag abfiltriert. Durch Einengen und nochmaliges Abkühlen konnte das Produkt vollständig abgetrennt werden.

Ausbeute: 1,94g (4,8 mmol) 49%, C₂₅H₃₃N₅, Schmelzpunkt: 54°C; ¹H NMR(CDCl₃): δ 1.19 (d 12H CH(CH₃)₂ J = 7 Hz), 2.05 (s 6H CH₃), 2.35 (s 3H CH₃), 2.73 (m 1H CH(CH₃)₂), 5.89-5.92 (m 4H Pyrrol), 7.93 (t 1H Pyridin), 8.47 (d 2H Pyrridin) MS: M⁺ = 403 m/z.

### [2,6-Diacetylpyridin-bis-(2-methyl-5-(2-methylphenyl))pyrrol-1-ylimin)] (26) :

2g (11 mmol) 1-Amino-2-methyl-5-(2-methylphenyl)pyrrol (16), 876mg (5,4 mmol) Diacetylpyridin und drei Tropfen Ameisensäure wurden in 30 ml Methanol 48 Stunden Rückfluß erhitzt. Anschließend wurde das Reaktionsgemisch auf 0°C abgekühlt, der entstandene Niederschlag abfiltriet und noch mehrmals mit kaltem Methanol gewaschen.

Ausbeute: 2,44g (4,9 mmol) 91%, C₃₃H₃₃N₅, Schmelzpunkt: 58°C; ¹H NMR(CDCl₃) : δ 2.13 (s 3H CH₃), 2.38 (s 3H CH₃), 2.56 (s 3H CH₃), 6.27 (m 2H Pyrrol), 6.35 (m 2H Pyrrol), 7.23-7.46 (m 8H Phenyl), 8.00 (t 1H Pyridin), 8.44 (d 2H Pyrridin) MS: M⁺ = 499 m/z.

### [2,6-Diacetylpyridin-bis-(2,5-diphenylpyrrol-1-ylimin)] (27) :

3g (12,8 mmol) ) 1-Amino-2,5-diphenylpyrrol (17) und 1,04g (6,4 mmol) Diacetylpyridin wurden 12 Stunden in Propionsäure Rückfluß erhitzt. Nach dem Erkalten der Reaktionsmischung wurde auf die Hälfte eingengt, der entstandene Niederschlag abfiltriert und mehrmals mit Methanol gewaschen.

Ausbeute: 0,75g (1,3 mmol) 10,2%, C₄₁H₃₃N₅, Schmelzpunkt: 228-231°C; ¹H NMR (CDCl₃) : δ 1.70 (s 6H Methyl), 6.44 (s 4H Pyrrol), 7.07-7.50 (m 20H Phenyl), 7.88 (m 1H Pyridin), 8.38 (m 2H Pyridin); MS: M⁺ = 595m/z.

### [2,6-Diacetylpyridin-bis-(2,3,5-triphenylpyrrol-1-ylimin)] (28) :

1,5g (4,8 mmol) 1-Amino-2,3,5-triphenylpyrrol (18) und 0,39g (2,4 mmol) Diacetylpyridin wurden in 20 ml Propionsäure 2 Tage Rückfluß erhitzt. Nach dem Erkalten und Abziehen des Lösungsmittels am Rotationsverdampfer wurde der Rückstand in Ethanol gelöst und bei -18 °C auskristallisiert. Durch Einengen und nochmaliges Auskristallisieren konnte das Produkt vollständig isoliert werden.

Ausbeute: 0,83g (1,1 mmol) 47%, C₅₃H₄₁N₅, Schmelzpunkt: 132°C; ¹H NMR(DMSO-d₆): δ 1.68 (s 6H Methyl), 6.75 (s 2H Pyrrol), 7.00-7.51 (m 30H Phenyl), 8.04 (m 1H Pyridin), 8.24 (m 2H Pyridin); MS: M⁺ = 747m/z.

### 2,6-Diacetylpyridin-bis-(2-methylindol-1-ylimin) (29) :

0,71g (4,9 mmol) 1-Amino-2-methylindol (19) (M.Somei, M.Natsume, Tetrahedron Lett., 5 (1974) 461) gelöst in 10 ml Methanol wurden mit 0,4g 2,4mmol) Diacetylpyridin versetzt und anschließend 12 Stunden Rückfluß erhitzt. Der entstandene Niederschlag wurde abfiltriert und mehrmals mit kaltem Methanol gewaschen.

Ausbeute: 0,75g (1,8 mmol) 73 %, C₂₇H₂₅N₅, Schmelzpunkt: 65°C, ¹H NMR(CDCl₃) : δ 2.43 (s 6H Methyl), 2.53 (s 6H Methyl), 6.43 (d 2H Pyrrol), 6.96-8.55 (m 11H Phenyl, Pyridyl); MS: M⁺ = 420 m/z.

### 2,6-Diacetylpyridin-di-carbazol-N-ylimin (30) :

2-3 g N-Aminocarbazols (20) (J.Klein, L. Davis, G. Olsen, G. Wong, F. Huger, J. Med. Chem., 39 (1996) 570) (2,2 Äquiv.) und ein Äquiv. Diacetylpyridin wurden in wenig Methanol (5 ml) gelöst und für 8 Stunden Rückfluß erhitzt (1 ml Ameisensäure wurde vorher zugegeben). Die Lösung ließ man erkalten und filtrierte das Diimin ab. Nach Trocknen am Hochvakuum erhielt man das Produkt in 75,1 % Ausbeute.

Schmelzpunkt: 184-188 °C; C₃₃H₂₅N₅; ¹H NMR (CDCl₃) : δ 2.61 (s 6H CH₃C=N), 7.95 (t 1H p Pyridin), 8.51 (d 2H m Pyridin), 7.2 (m 8H) 7.4 (m4H) 8.1 (m 4H) Carbazol; MS: M⁺ = 491 m/z.

### 2,6-Diacetylpyridin-bis-(3,5-dimethyl-1,2,4-triazol-4-ylimin) (31) :

960 mg 4-Amino-3,5-dimethyl-1,2,4-triazol (21) (R. Herbst, J. Garrison, J. Org. Chem., 18, (1953), 872-876) und 698 mg 2,6-Diacetylpyridin und 30 mg p-Toluolsulfonsäure wurden in 30 ml o-Dichlorbenzol für 8 Stunden Rückfluß erhitzt. Nach Erkalten fügte man langsam unter guter Rührung eine Mischung Hexan/Ether 1:1 zu um das Diimin auszufällen. Die überstehende Lösung wurde abdekantiert, das bräunliche Pulver noch einmal digeriert, erneut abdekantiert und dann am Hochvakuum getrocknet. 1,41 g (94,0 %), Schmelzpunkt: 252 °C Zersetzung; C₁₇H₂₁N₉; ¹H NMR (CD₃CN) : δ 2.25 (s 12H Methyl), 2.58 (s 6H CH₃C=N), 8.11 (t 1H p Pyridin), 8.45 (d 2H m Pyridin); MS: M⁺ = 351 m/z.

### 2,5-Diformylthiophen-bis-(2,5-diisopropylpyrrol-1-ylimin): (Figur 2) (32)

474 mg N-Amino-2,5-diisopropylpyrrol (13) und 200 mg 2,5-Diformylthiophen wurden in 10 ml Methanol gelöst und für 2 Stunden Rückfluß erhitzt (0,5 ml Ameisensäure wurden vorher zugegeben).

Die Lösung ließt man erkalten und filtriert das Diimin ab. Nach Trocknen am Hochvakuum erhält man 510 mg (81,8 %) Produkt.

Schmelzpunkt: 134-137 °C; C₂₆H₃₆N₄S; ¹H NMR (CDCl₃) : δ 1.00 (s 24H CH(CH₃), 2.81 (m 4H CH(CH₃)), 5.68 (s 4H Pyrrol), 7.12 (s 2H Thiophen), 8.24 (s 2H Formyl) ; MS: M⁺ = 437 m/z.

### e) Metallkomplexe (Figur 3) (34-52)

### [2,6-Diacetylpyridin-bis-(2,5-dimethylpyrrol-1-ylimin)]-FeCl₂ (34) :

In ein trockenens Schlenkrohr wurden 20 ml 2-Butanol gegeben und über eine Glasfritte 5 Minuten lang Argon eingeleitet bis das Lösungsmittel vollständig mit Argon gesättigt war. Dann wurden 200 mg 2,6-Diacetylpyridin-bis-(2,5-methylpyrrol-1-ylimin) (22) und 73 mg Eisen(II)chlorid zugegeben und für 1 Stunde auf 80°C erhitzt. Die Lösung färbte sich sofort dunkelgrün und es bildete sich ein Niederschlag mit derselben Farbe. Nach Erkalten ließ man den Niederschlag absitzen und dekantierte die überstehende Lösung ab. Der Rest an 2-Butanol wurde am Hochvakuum abgezogen, der Rückstand gepulvert und mehrmals mit absolutem Methylenchlorid extrahiert. Die grüne Methylenchloridphase wurde abgezogen, Rückstand 195 mg (72 %) ; C₂₁H₂₅N₅FeCl₂; Mr: 474,2 g/mol; NMR: paramagnetisch; MS: M⁺ = 474 m/z.

### [2,6-Diacetylpyridin-bis-(2,5-dimethylpyrrol-1-ylimin)]-FeCl₃ (35) :

In ein trockenens Schlenkrohr wurden 20 ml absolutes Methylenchlorid, 367 mg 2,6-Diacetylpyridin-bis-(2,5-methylpyrrol-1-ylimin) (22) und 175 mg Eisen(III)chlorid gegeben; die Lösung färbte sich sofort dunkelbraun und es bildete sich ein Niederschlag mit derselben Farbe. Nach 15 Stunden Rühren bei Raumtemperatur wurde das Methylenchlorid am Hochvakuum abgezogen und der braune Rückstand zwei mal mit absolutem Hexan (10 ml) digeriert und jeweils abdekantiert. Das so erhaltene Produkt wurde am Hochvakuum getrocknet. Ausbeute: 420 mg (78.0 %) , C₂₁H₂₅N₅FeCl₃; Mr: 509,6 g/mol; NMR: para-magnetisch.

### [2,6-Diacetylpyridin-bis-(2,5-dimethylpyrrol-1-ylimin)]-CoCl₂ (36) :

In ein trockenens Schlenkrohr wurden 10 ml 2-Butanol gegeben und über eine Porzellanfritte 5 Minuten lang Argon eingeleiten bis das Lösungsmittel. vollständig mit Argon gesättigt war. Dann wurden 150 mg 2,6-Diacetylpyridin-bis-(2,5-methylpyrrol-1-ylimin) (22) und 56 mg Kobalt(II)chlorid zugegeben und für 1 Stunde auf 80° C erhitzt. Die Lösung färbte sich sofort dunkelbraun und es bildete sich ein Niederschlag mit derselben Farbe. Nach Erkalten ließ man den Niederschlag absitzen und dekantierte die überstehende Lösung ab. Der Rest an 2-Butanol wurde am Hochvakuum abgezogen, der Rückstand gepulvert. 155 mg (75,2 %); C₂₁H₂₅N₅CoCl₂; Mr: 477,3 g/mol; NMR: paramagnetisch.

### [2,6-Diacetylpyridin-bis-(2,5-diisopropylpyrrol-1-ylimin)]-FeCl₂ (37) :

30 ml 2-Butanol wurden in ein trockenes Schlenkrohr gegeben und für 5 Minuten mit Argon gespült. Nach der Zugabe von 1.0 g 2,6-Diacetylpyridin-bis-(2,5-diisopropylpyrrol-1-ylimin) (23) und 274 mg Eisen(II)chlorid wurde die dunkelgrüne Lösung für 2 Stunden bei 80° C gerührt. Das Lösungsmittel wurde dann am Hochvakuum abgezogen, der Rückstand gepulvert und mehrmals mit 100 ml absolutem Methylenchlorid extrahiert. Nach Abziehen von Mehylenchlorid blieben 1,04 g (81,5 %) grünes Pulver zurück. C₂₉H₄₁N₅FeCl₂; Mr: 586,4 g/mol; NMR: paramagnetisch.

### [2,6-Diacetylpyridin-bis-(2,5-diisopropylpyrrol-1-ylimin)]-FeCl₃ (38) :

In ein trockenens Schlenkrohr wurden 20 ml absolutes Methylenchlorid, 400 mg 2,6-Diacetylpyridin-bis-(2,5-diisopropylpyrrol-1-ylimin) (23) und 141 mg Eisen(III)chlorid gegeben; die Lösung färbte sich sofort dunkelbraun. Nach 15 Stunden Rühren bei Raumtemperatur wurde das Methylenchlorid am Hochvakuum abgezogen und der braune Rückstand zwei mal mit absouten Hexan (10 ml) digeriert und jeweils abdekantiert. Das so erhaltene Produkt wurde am Hochvakuum getrocknet. Ausbeute: 461 mg (85,2 %), C₂₉H₄₁N₅FeCl₃, Mr: 621,9 g/mol, NMR: paramagnetisch.

### [2,6-Diacetylpyridin-bis-(2,5-diisopropylpyrrol-1-ylimin)]-CoCl₂ (39) :

In ein trockenens Schlenkrohr wurden 50 ml absolutes Tetrahydrofuran, 500 mg 2,6-Diacetylpyridin-bis-(2,5-diisopropylpyrrol-1-ylimin) (23) und 141 mg Kobalt(II)chlorid gegeben. Die Lösung färbte sich sofort dunkelbraun und es bildete sich ein Niederschlag mit derselben Farbe. Nach 12 Stunden Rühren wurde das Lösungsmittel am Hochvakuum abgezogen, der braune Rückstand zweimal mit absolutem Hexan digeriert, jeweils von der überstehenden Lösung abdekantiert und getrocknet. 450 mg (70,2 %) ; C₂₉H₄₁N₅CoCl₂; Mr: 589,5 g/mol.

### [2,6-Diacetylpyridin-bis-(2-methyl-5-phenylpyrrol-1-ylimin)]-FeCl₂ (40) :

Eine Lösung von 0,14g (1,1 mmol) Eisen(II)chlorid und 0,48g (1,0 mmol) 2,6-Diacetylpyridin-bis-(2-methyl-5-phenylpyrrol-1-ylimin) (24) wurden in 30 ml 2-Butanol bei 40°C über Nacht gerührt. Der entstandene Niederschlag wurde unter Argon abfiltriert und mehrmals mit absolutem Ether gewaschen.

Ausbeute: 0,23g (0,4 mmol) 37%, C₃₁H₂₉FeCl₂N₅, Schmelzpunkt: >350°C (Zers.); IR: KBr (cm⁻¹) 3071(w), 2919(w), 1640(s), 1605(s), 1514(m), 1445(m), 1373(s), 1298(w), 1273(w), 1204(w), 1074(w), 1026(w), 810(w), 756(s), 698(m), 605(w), 565(w), 497(w).

### [2,6-Diacetylpyridin-bis-(2-methyl-5-phenylpyrrol-1-ylimin)]-CoCl₂ (41) :

0,41g (0,9 mmol) Diacetylpyridin-bis-(2-methyl-5-phenylpyrrol-1-ylimin) (24) und 0,12g (0,9 mmol) Kobalt(II)chlorid wurden in 30 ml THF gelöst und anschließend zwölf Stunden bei Raumtemperatur gerührt. Durch vollständige Ausfällung mit Ether und mehrmaliges Waschen des filtrierten Produktes mit Ether kann die Verbindung analysenrein erhalten werden.

Ausbeute: 0,29g (0,5 mmol) 54%, C₃₁H₂₉Cl₂CoN₅, Schmelzpunkt: >350°C (Zers.); IR: KBr (cm⁻¹) 3071(w), 3031(w), 2917(w), 1601(m), 1576(m), 1514(s), 1474(w), 1445(m), 1375(s), 1331(s), 1300(w), 1271(m), 1205(w), 1180(w), 1026(m), 810(w), 752(s), 729(m), 698(s); MS: M⁺ - Cl = 566 m/z.

### [2,6-Diacetylpyridin-bis-(2-methyl-5-isopropylpyrrol-1-ylimin))-FeCl₂ (42) :

Eine Lösung von 0,27g (2,1 mmol) Eisen(II)chlorid und 0,86g (2,1 mmol) 2,6-Diacetyl-pyridin-bis-(2-methyl-5-isopropylpyrrol-1-ylimin) (25) wurden in 150 ml 2-Butanol bei 40°C über Nacht gerührt. Der entstandene Niederschlag wurde unter Argon abfiltriert und mehrmals mit absolutem Ether gewaschen.

Ausbeute: 0,82g (1,5 mmol) 72%, C₂₅H₃₃FeCl₂N₅, Schmelzpunkt: >350°C (Zers.); IR: KBr (cm⁻¹) 2963(s), 2925(s), 2871(m), 1615(w), 1578(s), 1449(w), 1402(s), 1373(s), 1362(m), 1267(s), 1223(w), 1202(m), 1105(w), 1026(m), 812(m), 748(s).

### [2,6-Diacetylpyridin-bis-(2-methyl-5-isopropylpyrrol-2-ylimin)]-CoCl₂ (43) :

0,8g (2,0 mmol) Diacetylpyridin-bis-(2-methyl-5-isopropylpyrrol-1-ylimin) (25) und 0,26g (2,0 mmol) Kobalt(II)chlorid wurden in 15 ml THF suspendiert und anschließend zwölf Stunden bei Raumtemperatur gerührt. Durch vollständige Ausfällung mit Ether und mehrmaliges Waschen des filtrierten Produktes mit Ether kann die Verbindung analysenrein erhalten werden.

Ausbeute: 0,99g (1,9 mmol) 94%, C₂₅H₃₃Cl₂CoN₅, Schmelzpunkt: >350°C (Zers.) ; IR: KBr (cm⁻¹) 2963(s), 2925(m), 2871(m), 1624(m), 1578(s), 1447(w), 1402(s), 1373(s), 1362(s), 1333(w), 1269(s), 1204(w), 1103(w), 1026(m), 812(m), 748(s); MS: M⁺ - Cl = 498 m/z.

### [2,6-Diacetylpyridin-bis-(2-methyl-5-(2-methylphenyl))pyrrol-1-ylimin)]-FeCl₂ (44) :

0,98g (2 mmol) 2,6-Diacetylpyridin-bis-(2-methyl-5-(2-methylphenyl)pyrrol-1-ylimin) (26) und 0,25g (2 mmol) Eisen(II)chlorid wurden in 40 ml Argon gesättigtem 2-Butanol 12 Stunden bei 40°C gerührt. Anschließend wurde der entstandene Niederschlag abfiltriert und mehrmals mit Ether gewaschen.

Ausbeute: 0,80g (1,3 mmol) 65%, C₃₃H₃₃FeCl₂N₅, Schmelzpunkt: >350°C (Zers.); IR: KBr (cm⁻¹) 2952 (w), 2921(w), 1624(w), 1603(w), 1578(w), 1516(m), 1479(w), 1437(m), 1373(s), 1294(m), 1283(m), 1202(w), 1028(w), 806(m), 754(s), 733(m).

### [2,6-Diacetylpyridin-bis-(2-methyl-5-(2-methylphenyl))pyrrol-1-ylimin)]-CoCl₂ (45) :

1,4g (2,8 mmol) 2,6-Diacetylpyridin-bis-(2-methyl-5-(2-methylphenyl))pyrrol-1-ylimin) (26) und 0,36g (2,8 mmol) Kobalt(II)chlorid wurden 12 Stunden bei Raumtemperatur gerührt. Der enstandene braune Niederschlag wurde abfiltriert und mehrmals mit Ether gewaschen. Der in THF gelöste Teil des Komplexes wurde anschließend mit Ether vollständig ausgefällt und abermals abfiltriert.

Ausbeute: 0,69g (1,1 mmol) 39%, C₃₃H₃₃Cl₂CoN₅, Schmelzpunkt: >350°C (Zers.); IR: KBr (cm⁻¹) 2921(w), 1626(m), 1578(m), 1514(m), 1479(w), 1437(w), 1373(s), 1327(w), 1296(m), 1269(w), 1204(m), 1124(w), 1099(w), 1026(m), 810(w), 756(s), 733(m), MS: M⁺ - Cl = 593 m/z.

### [2,6-Diacetylpyridin-bis-(2-methylindol-1-ylimin)]-FeCl₂ (46) :

Zu einer Lösung von 0,12g (0,1 mmol) Eisen(II)chlorid in 50 ml 2-Butanol wurden 0,4g (0,1 mmol) 2,6-Diacetylpyridin-bis-(2-methylindol-1-ylimin) (29) gelöst in 100 ml 2-Butanol langsam zugetropft. Nach zwei Stunden Rühren bei 40°C wurde der entstandene Niederschlag unter Argon abfiltriert und anschließend mehrmals mit Ether digeriert.

Ausbeute: 277mg (0,5 mmol) 53 %, C₂₇H₂₅Cl₂FeN₅, Schmelzpunkt: > 350°C (Zers.), IR (KBr) cm⁻¹: 2917(w), 1609(m), 1574(m), 1555(w), 1476(w), 1452(s), 1373(s),11344(m), 1321(m), 1306(m), 1271(m), 1225(s), 775(m), 742(s).

### [2,6-Diacetylpyridin-bis-(2-methylindol-1-ylimin)]-CoCl₂ (47) :

0,29g (0,67 mmol) 2,6-Diacetylpyridin-bis-(2-methylindol-1-ylimin) (29) und 88mg (0,67 mmol) Kobalt(II)chlorid wurden in 50 ml THF gelöst und anschließend zwölf Stunden bei Raumtemperatur gerührt. Durch vollständige Ausfällung mit Ether, Filtration und mehrmaliges Waschen mit Ether kann das Produkt analysenrein isoliert werden.

Ausbeute: 217mg (0,40 mmol) 58 %, C₂₇H₂₅Cl₂CoN₅, Schmelzpunkt: > 350°C (Zers.), IR (KBr) cm⁻¹: 3054(w), 2917(w), 1609(m), 1576(m), 1553(w), 1452(s), 1373(s), 1348(s), 1321(s), 1306(m), 1269(m), 1227(m), 1205(w), 1028(w), 1014(w), 812(m), 775(m), 742(s); MS: M⁺ -Cl = 514 m/z.

### (2,6-Diacetylpyridin-di-carbazol-N-ylimin)-FeCl₂ (48) :

15 ml 2-Butanol wurden in ein trockenes Schlenkrohr gegeben und für 5 Minuten mit Argon gespült. Nach der Zugabe von 240 mg 2,6-Diacetylpyridindicarbazol-N-ylimin (30) und 78 mg Eisen(II)chlorid wurde die dunkelbraune Lösung für 2 Stunden bei 80° C gerührt. Nach dem Erkalten wurden das Lösungsmittel abdekantiert und der braune Rückstand am Hochvakuum getrocknet. Ausbeute: 151 mg (49,6 %) C₃₃H₂₅N₅FeCl₂; Mr: 618,4 g/mol.

### (2,6-Diacetylpyridin-di-carbazol-N-ylimin)-CoCl₂ (49) :

In 20 ml absolutem Tetrahydrofuran wurden 240 mg 2,6-Diacetylpyridindicarbazol-N-ylimin (30) und 78 mg Eisen(II)chlorid gegeben. Die dunkelbraune Lösung wurde 12 Stunden bei Raumtemperatur gerührt und anschließend wurde das Lösungsmittel am Hochvakuum abgezogen. Ausbeute: 110 mg (87,3 %) C₃₃H₂₅N₅CoCl₂; Mr: 621,4 g/mol.

### [2,6-Diactylpyridin-bis-(3,5-dimethyl-1,2,4-triazol-4-ylimin)]FeCl₂ (50) :

250 mg 2,6-Diactylpyridin-bis-(2,5-dimethyl-1,2,4-triazol-4-ylimin) (31) und 450 mg Eisen (II) Chlorid (5 Äquiv.) wurden in 100 ml absolutem Ethanol gelöst beziehungsweise suspendiert. Die blaue Lösung wurde für 12 Stunden bei 50 °C gerührt und dann das Lösungsmittel am Hochvakuum abgezogen. Violetter Rückstand, 700 mg.

### [2,5-Diformylthiophen-bis-(2,5-diisopropylpyrrol-1-ylimin)]-CoCl₂ (51) :

20 ml 2-Butanol wurden in ein trockenes Schlenkrohr gegeben und für 5 Minuten mit Argon gespült. Nach der Zugabe von 250 mg 2,5-Diformylthiophen-bis-(2,5-diisopropylpyrrol-1-ylimin) (32) und 73 mg Kobalt(II)chlorid wurde die dunkelgrüne Lösung für 1 Stunden bei 80° C gerührt. Das Lösungsmittel wurde dann am Hochvakuum abgezogen und der Rückstand gepulvert. Ausbeute: 285 mg (93,2 %) grünes Pulver. C₂₆H₃₆N₅SCoCl₂; Mr: 566,5 g/mol.

### f) Polymerisationen

### Ethylenpolymerisation

### Standardpolymerisationsverfahren

In einem inertisierten Kolben mit mechanischem Rührer und Ethyleneinleitungsrohr wurden 150 ml (bzw. 250 ml) Toluol vorgelegt. Dazu wurden soviele ml einer 30%igen Lösung von Methylaluminoxan (MAO) in Toluol gegeben, daß man, bezogen auf den später zugesetzten Katalysatorkomplex, 100 Äquivalente einsetzte. gegebenenfalls wurden 12,5 ml (bzw. 25 ml) 1-Hexen zugegeben. Dann wurden 50 mmol (bzw. 100 mmol) des zu untersuchenden Komplexes dazugegeben. Es wurde ein Ethylenstrom von 40 l/h durch die Reaktionslösung geleitet und eine Temperatur von 30°C eingestellt. Nach 1 h wurde die Reaktion durch Zugabe eines Gemisches aus 15 ml konzentrierter Salzsäure und 50 ml Methanol gestoppt. Wenn ein Niederschlag zu beobachten war, wurde der Rückstand gewaschen und anschließend unter leichtem Vakuum getrocknet. War kein Niederschlag zu beobachten, wurden die Phasen getrennt und die polare Phase mit 100 ml Toluol ausgeschüttelt. Die organischen Phasen wurden vereinigt und die flüchtigen Bestandteile am Rotationsverdampfer unter Vakuum entfernt.

In den Tabellen 1 und 2 sind Einzelheiten zu den Polymerisationen von Ethylen mit Eisen- (Tabelle 1) bzw. Cobalt-Katalysatoren (Tabelle 2) dargestellt. Die Tabellen 3 und 4 zeigen die Polymeranalytik des entstandenen Polyethylens (Tabelle 3: Polyethylen mittels Eisenkatalysatoren, Tabelle 4: Polyethylen mittels Cobaltkatalysatoren)

**Tabelle 1:**

| Ethylenpolymerisation mit Eisen-Katalysatoren | | | | | |
|---|---|---|---|---|---|
| Polymerisation | Katalysator | Ansatzgröße [µmol] | Comonomer | Polymeraustrag [g] | Aktivität¹ [gPE/ mmol-Kat.xh] |
| V²1 | V³ | 100 | | 32 | 320 |
| V²2 | V³ | 100 | Hexen | 14,5 | 145 |
| 1 | 37 | 100 | | 19 | 190 |
| 2 | 37 | 50 | Hexen | 20,2 | 404 |
| 3 | 34 | 100 | | 56,5 | 565 |
| 4 | 34 | 100 | Hexen | 69 | 690 |
| 5 | 48 | 50 | | 16 (Öl) | 320 |
| 6 | 48 | 50 | Hexen | 46 (Öl) | 920 |
| 7 | 46 | 50 | | 16 | 320 |
| 8 | 46 | 50 | Hexen | 15,5 | 310 |
| 9 | 42 | 50 | | 4 | 80 |
| 10 | 42 | 50 | Hexen | 49 | 980 |
| 11 | 40 | 50 | | 3,6 | 72 |
| 12 | 40 | 50 | Hexen | 3,7 | 74 |
| 13 | 44 | 50 | | 1,5 | 30 |
| 14 | 44 | 50 | Hexen | 4,8 | 96 |
| 15 | 35 | 50 | | 1,0 | 20 |

| | | | | | |
|---|---|---|---|---|---|
| ¹ Bei Ölen bezogen auf die isolierbare Ausbeute | | | | | |
| ² Vergleichsversuch | | | | | |
| ³ 2,6-Diacetylpyridein-bis-(2,6-dimethylphenylimin)-FeCl₂ | | | | | |

**Tabelle 2:**

| Ethylenpolymerisation mit Cobalt-Katalysatoren | | | | | |
|---|---|---|---|---|---|
| Polymerisation | Katalysator | Ansatzgröße [µmol] | Comonomer | Polymeraustrag [g] | Aktivität¹ [gPE/ mmol-Kat.xh] |
| 16 | 36 | 100 | | 1 | 10 |
| 17 | 36 | 100 | Hexen | 24 | 240 |
| 18 | 39 | 100 | | 5 | 50 |
| 19 | 39 | 100 | Hexen | 0,6 | 6 |
| 20 | 49 | 50 | | 2 (Öl) | 40 |
| 21 | 49 | 50 | Hexen | 2,5 (Öl) | 50 |
| 22 | 51 | 50 | | 0,2 (Öl) | 4 |
| 23 | 51 | 50 | Hexen | 0,1 (Öl) | 2 |
| 24 | 47 | 50 | | 0,2 | 4 |
| 25 | 47 | 50 | Hexen | 0,6 | 12 |
| 26 | 43 | 50 | | 8,6 | 172 |
| 27 | 43 | 50 | Hexen | 11,5 | 230 |
| 28 | 41 | 50 | | 11,2 (Öl) | 224 |
| 29 | 41 | 50 | Hexen | 24,2 (Öl) | 482 |
| 30 | 45 | 50 | | 0,6 (Öl) | 12 |
| 31 | 45 | 50 | Hexen | 1,5 (Öl) | 30 |

| | | | | | |
|---|---|---|---|---|---|
| ¹ Bei Ölen bezogen auf die isolierbare Ausbeute | | | | | |

**Tabelle 3:**

| Polymeranalytik Polyethylen (Eisen-Katalysatoren) | | | | | | |
|---|---|---|---|---|---|---|
| Polymerisation | eta-Wert [dl/g] | DSC₁ [°C] | M_{w}² | Mn³ | M_{w}/Mn | Dichte [g/cm³] |
| V⁴1 | 2,72 | 135 | 318385 | 7036 | 45 | 0,9596 |
| V⁴2 | 2,71 | 138 | 245448 | 4172 | 59 | 0,9625 |
| 1 | 1,6 | 131 | 112907 | 6483 | 17,4 | 0,9600 |
| 2 | 2,45 | 131 | 205544 | 9298 | 22,1 | 0,9541 |
| 3 | 0,1 | 118 | 3401 | 1273 | 2,7 | 0,9241 |
| 4 | 0,1 | 117 | 3251 | 1440 | 2,3 | 0,9283 |
| 7 | 0,06 | 63 | 1509 | 727 | 2,1 | 0,8913 |
| 8 | 0,12 | 63 | 1477 | 839 | 1,8 | 0,9061 |
| 9 | 0,44 | 120 | 14084 | 1158 | 12,2 | 0,9177 |
| 10 | 0,61 | 121 | 29937 | 1849 | 16,2 | 0,924⁵ |
| 11 | 0,22 | 121 | 5438 | 1320 | 4,1 | 0,9338 |
| 12 | 0,21 | 119 | 5956 | 1224 | 4,9 | 0,9317 |
| 13 | 0,31 | 123 | 8183 | 1329 | 6,2 | 0,9387 |
| 14 | 0,18 | 119 | 4737 | 1356 | 3,5 | 0,9387 |
| 15 | 0,1 | 117 | 2281 | 847 | 2,7 | |

| | | | | | | |
|---|---|---|---|---|---|---|
| ¹ Differentialthermoanalyse | | | | | | |
| ² gewichtsmittleres Molekulargewicht | | | | | | |
| ³ zahlenmittleres Molekulargewicht | | | | | | |
| ⁴ Vergleichsversuch | | | | | | |
| ⁵ Hexengehalt: 2,4 mol-% | | | | | | |

**Tabelle 4:**

| Polymeranalytik Polyethylen (Cobalt-Katalysatoren) | | | | | | |
|---|---|---|---|---|---|---|
| Polymerisation | eta-Wert [dl/g] | DSC₁ [°C] | M_{w}² | Mn³ | M_{w}/Mn | Dichte [g/cm³] |
| 16 | 0,1 | 118 | 3582 | 1000 | 3,6 | 0,9271 |
| 17 | 0,05 | 83 | 879 | 659 | 1,3 | 0,9247 |
| 18 | 0,45 | 127 | 13600 | 3181 | 4,3 | 0,9605 |
| 19 | 0,48 | 126 | 9564 | 2735 | 3,5 | 0,9721 |
| 24 | 0,13 | 120 | 5896 | 1478 | 4,0 | 0,9333 |
| 25 | 0,1 | 65 | 1323 | 719 | 1,8 | 0,8948 |
| 26 | 0,27 | 124 | 4985 | 1949 | 2,6 | 0,9563 |
| 27 | 0,17 | 123 | 4565 | 1796 | 2,5 | 0,9528 |

| | | | | | | |
|---|---|---|---|---|---|---|
| ¹ Differentialthermoanalyse | | | | | | |
| ² gewichtsmittleres Molekulargewicht | | | | | | |
| ³ zahlenmittleres Molekulargewicht | | | | | | |

Die Polymerisate der Polymerisationen 3 und 4 sowie der Polymerisationen 5 und 6 wurden NMR-spektroskopisch (mittels ¹H- und ¹³C NMR-Spektroskopie) genauer untersucht. Die Tabellen 5 bzw. 6 zeigen die entsprechenden Daten.

**Tabelle 5:**

| NMR-spektroskopisch ermittelte Daten der Polymerisate der Polymerisationen 3 und 4: | | | | | | |
|---|---|---|---|---|---|---|
| Verzweigungstypen (in Methylendgruppen/1000C) | | | | | | |
| Polymerisation | Σ Methyl-verzw.¹ | Σ Ethyl-verzw.¹ | Σ Butyl-verzw.¹ | Σ Pentyl-verzw.¹ | Σ >C₆-ver zw.¹ | Gesamtsumme Me/1000 C² |
| 3 | 3,5 | 0,8 | 0 | 3,4 | 15,9 | 23,6 |
| 4³ | 6,9 | 1,9 | 9,1 | 6,2 | 26,8 | 50,9 |

| | | | | | | |
|---|---|---|---|---|---|---|
| ¹ Summe der entsprechenden Verzweigungen | | | | | | |
| ² Gesamtsumme an Methylendgruppen pro 1000 C-Atome | | | | | | |
| ³ Die Probe enthält 1,8 mol-% Hexen | | | | | | |

| Doppelbindungen/1000C | | |
|---|---|---|
| Polymerisation | VinylDB⁴ | transDB⁴ |
| 3 | 18 | 1,7 |
| 4 | 8,5 | 10,1 |

| | | |
|---|---|---|
| 4 Anzahl Doppelbindungen pro 1000 C-Atome | | |

**Tabelle 6:**

| NMR-spektroskopisch ermittelte Daten der Polymerisate der Polymerisationen 5 und 6: | | | | | |
|---|---|---|---|---|---|
| Verzweigungstypen (in Methylendgruppen/1000C) | | | | | |
| Polymerisation | Σ Methyl-verzw.¹ | Σ Ethyl-verzw.¹ | **Σ** Butyl-verzw.¹ | **Σ** -C₆-Verzw .¹ | Gesamtsumme Me/1000C² |
| 5 | 19 | 71 | 11 | 80 | 181 |
| 6 | 17 | 71 | 15 | 79 | 182 |

| | | | | | |
|---|---|---|---|---|---|
| ¹ Summe der entsprechenden Verzweigungen | | | | | |
| ² Gesamtsumme an Methylendgruppen pro 1000 C-Atome | | | | | |

| Doppelbindungen/1000C | | | | |
|---|---|---|---|---|
| Polymerisation | Vinyl | Vinyliden | cis/ trans | CH=C< |
| 5 | 1,1 | 0,2 | 4,3 | 0,6 |
| 6 | 1,1 | 0,2 | 4,8 | 0,4 |

### Oligomere: Polymerisation von Ethylen mit Komplex 20

Nach der Polymerisation wurde die Reaktionslösung mit Methanol/HCl aufgearbeitet und die wäßrige Phase von der Toluolphase getrennt. Ein kleiner Teil der organischen Phase wurde unbehandelt bezüglich der Zusammensetzung untersucht. Der Rest wurde aufgearbeitet, indem das Lösungsmittel im Vakuum entfernt wurde. Man erhält 2g eines Öls. Sowohl die unbehandelte Phase als auch das Öl wurden gaschromatographisch untersucht. Tabelle 7 zeigt die ermittelte Verteilung der Kohlenstoffketten.

### Einwaage toluolische Lösung: 0,3892g, Einwaage Öl: 0,2467g

**Tabelle 7:**

| Verteilung in mg in der Probe: | | | | | | | | | |
|---|---|---|---|---|---|---|---|---|---|
| Probe | <C₆ | C₆ | C₈ | C₁₀ | C₁₂ | C₁4 | C₁6 | C₁8 | >C₁8 |
| Lösung | 3 | 7,1 | 9,8 | 7,2 | 4 | 1,9 | 0,9 | 0,4 | 0,4 |
| Öl | | | | | 14,4 | 67,4 | 73,2 | 45,1 | 36,6 |
| Selektivitäten in % | | | | | | | | | |

| Probe | <C₆ | C₆ | C8 | C₁₀ | C₁₂ | C₁4 | C₁6 | C₁8 | >C₁8 |
|---|---|---|---|---|---|---|---|---|---|
| Lösung | 8,6 | 20,5 | 28,5 | 20,7 | 11,5 | 5,5 | 2,6 | 1,2 | 1,2 |
| Öl | | | | | 6,1 | 28,5 | 30,9 | 19 | 15,5 |

Es ist offensichtlich, daß bei der Aufarbeitung der Probe die niedermolekularen, leicht flüchtigen Anteile zusammen mit dem Lösungsmittel entfernt wurden. Die nicht aufgearbeitete Probe besitzt praktisch keine Bestandteile, die mehr als 18 Kohlenstoffatome aufweisen.

### Propenpolymerisation

Die Propenpolymerisation wurde analog der Ethylenpolymerisation durchgeführt, wobei Propylen statt Ethylen verwendet wurde.

Katalysator: Komplex 34, Ansatzgröße: 50 mmol, Polymeraustrag: 15g (Öl), Aktivität (gPE/mmolKatxh): 300.

### g) Trägerkatalysatoren:

### Trägerherstellung

Zu einer Suspension von 160 g Kieselgel (ES70X, Crosfield) in 1500 ml Heptan wurden innerhalb von 1 h 170 ml einer 2 molaren Lösung von Trimethylaluminium in Heptan getropft. Es wurde für 1 h gerührt und anschließend abfiltriert. Der Rückstand wurde zweimal mit Heptan gewaschen und dann im Vakuum getrocknet.

### Beladung

Zu einer Suspension von 10 g des Hergestellten Trägers in 35 ml Toluol wurden 119 mg des Komplexes 34 und 6,8 ml Methylaluminoxan (MAO; 30%-ige Lösung in Toluol) zugegeben. Man ließ 30 min. rühren, filtriert ab, wusch den Rückstand zweimal mit Toluol und trocknete anschließend den Rückstand im Vakuum. Man erhielt den Katalysator als rieselfähiges Pulver.

### Polymerisation

In einem gerührten 11-Stahlautoklaven wurden nach sorgfältigem Spülen mit Stickstoff und Temperieren auf die Polymerisationstemperatur von 70°C 450 ml iso-Butan und 60 mg MAO (als 30%-ige Lösung in Toluol) vorgelegt. Dann wurden 176 mg des geträgerten Katalysators mit weiteren 50 ml iso-Butan eingespült und Ethylen auf einen Gesamtdruck von 38 bar aufgepreßt. Der Druck im Autoklaven wurde durch Nachdosierung von Ethylen konstant gehalten. Nach 90 min. wurde die Polymerisation durch Entspannen der Polymerisationsmischung im Autoklaven abgebrochen. Man erhält 120 g Polyethylen; eta-Wert: 0,6 dl/g.

In den folgenden Figuren zeigt:
- Figur 1:: 1,4-Diketone: 1 - 7, N-Acetamid-Pyrrole: 8 - 11, N-Amino-Heterocyclen: 12 - 21; Me = Methyl, i-Pr = Isopropyl, Ph = Phenyl, o-Tol = ortho-Tolyl, R = Me
- Figur 2:: Liganden: 22 - 32; Me = Methyl, i-Pr = Isopropyl, Ph = Phenyl, o-Tol = ortho-Tolyl
- Figur 3:: Metallkomplexe: 34 - 52; Me = Methyl, i-Pr = Isopropyl, Ph = Phenyl, o-Tol = ortho-Tolyl

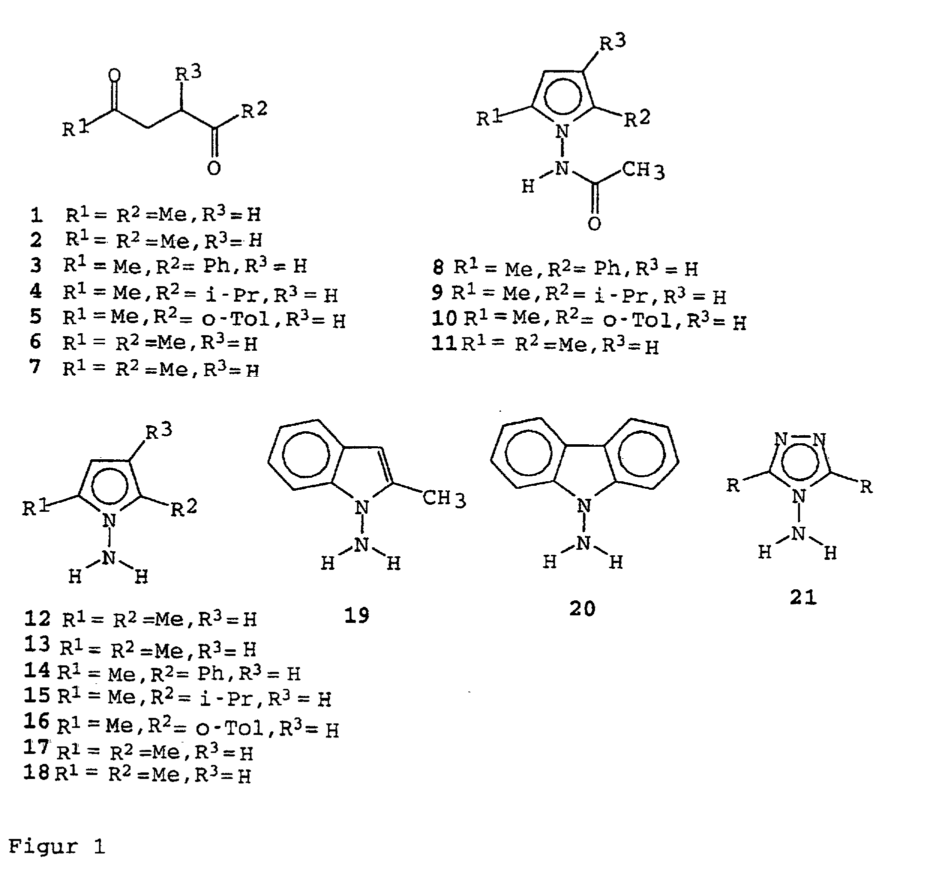

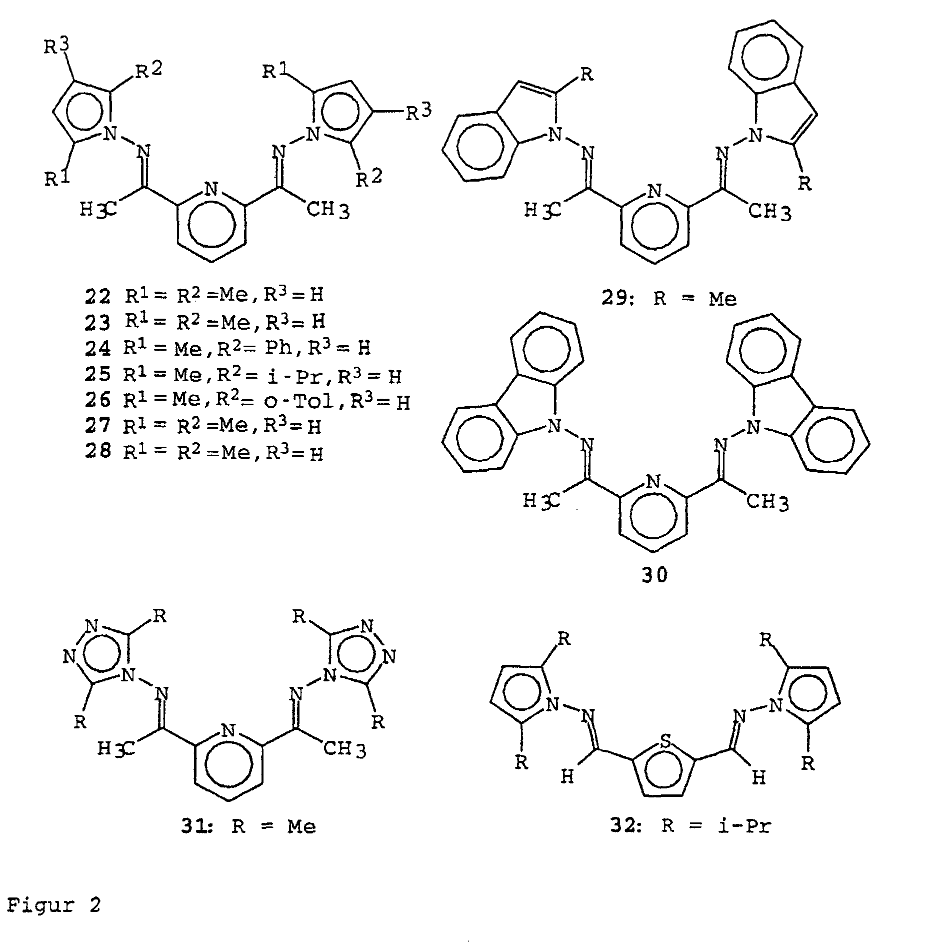

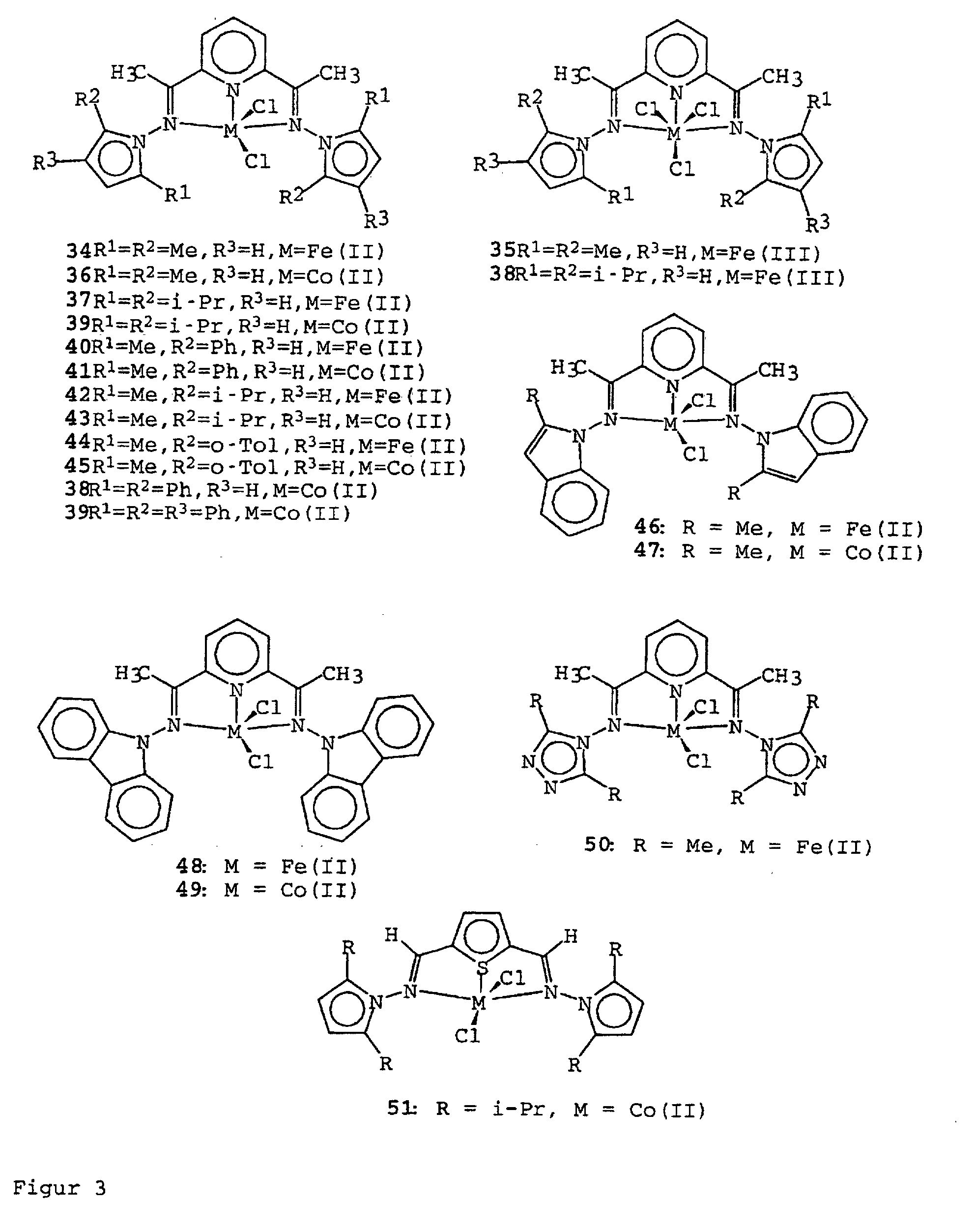

## Patentansprüche

1. Verbindungen der allgemeinen Formel (I) 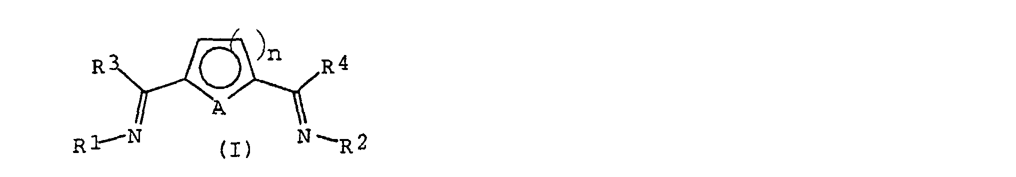 in der die Symbole die folgende Bedeutung haben:
A Nichtmetall ausgewählt aus N, S, O und P
R¹ Reste der allgemeinen Formel NR⁵R⁶,
R² Reste der allgemeinen Formel NR⁵R⁶ oder NR⁷R⁸, Alkyl-, Aryl- oder Cycloalkylreste,
R⁵ und R⁶ bilden gemeinsam mit dem N-Atom einen 5-, 6- oder 7-gliedrigen Ring, in dem eine oder mehrere der -CH- oder -CH₂-Gruppen durch geeignete Heteroatomgruppen ersetzt sein können, der gesättigt, ungesättigt und unsubstituiert, substituiert oder mit weiteren carbacyclischen oder heterocarbacyclischen 5- oder 6-gliedrigen Ringen, die ihrerseits gesättigt oder ungesättigt und substituiert oder unsubstituiert sein können, anelliert sein kann, und
R⁷ und R⁸ unabhängig voneinander Alkyl-, Aryl- oder Cycloalkylreste,
und
R³, R⁴ unabhängig voneinander H, Alkyl-, Aryl- oder Cycloalkylreste,
und
n 1 oder 2.

2. Verbindungen nach Anspruch 1, **dadurch gekennzeichnet, daß** die Reste der allgemeinen Formel NR⁵R⁶ Pyrrol- oder von Pyrrol abgeleitete Reste, worin eine oder mehrere -CH-Gruppen im Pyrrolring durch Stickstoff ausgetauscht sein können, sind, die unsubstituiert, substituiert oder mit weiteren carbacyclischen oder heterocarbacyclischen 5- oder 6-gliedrigen Ringen, die ihrerseits gesättigt oder ungesättigt und substituiert oder unsubstituiert sein können, anelliert sein können.

3. Verbindungen nach Anspruch 2, **dadurch gekennzeichnet, daß** die Pyrrol- oder von Pyrrol abgeleiteten Reste in der 2- und 5-Position mit C₁- bis C₆-Alkylgruppen, die linear, verzweigt und mit Heteroatomen substituiert sein können, und/oder Arylgruppen, die unsubstituiert oder ihrerseits mit Heteroatomen oder C₁- bis C₆-Alkylgruppen, die heteroatomsubstituiert sein können, substituiert sind.

4. Verbindungen nach Anspruch 1 und 2, **dadurch gekennzeichnet, dass** die Pyrrolreste oder von Pyrrol abgeleiteten Reste in der 2- oder 5-Position substituiert sind mit elektronenziehenden Resten, ausgewählt aus
- Halogen,
- NO₂,
- Sulfonate, ausgeählt aus
- SO₃R*,
- SO₃SiR*₃ oder
- SO₃⁻ (H-NR*₃)⁺
- -Trihalogenmethyl,
wobei R* gleich oder verschieden sein können und ausgewählt wird aus H, C₁-C₁₀-ALkyl, C₆-C₂₀-Aryl oder C₅-C₈-Cycloalkyl.

5. Verbindungen nach einem der Ansprüche 1 bis 4, **dadurch gekennzeichnet, daß** in der allgemeinen Formel (I) gemäß Anspruch 1 A = N und n = 2 ist.

6. Verbindungen nach Anspruch 5, **dadurch gekennzeichnet, daß** sie einer der allgemeinen Formeln (Ia), (Ib), (Ic) oder (Id) : 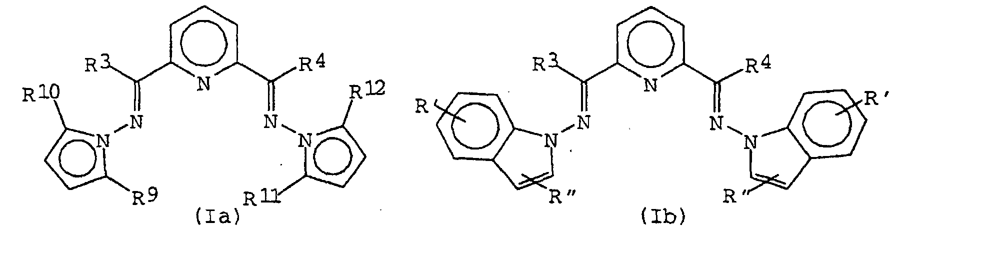 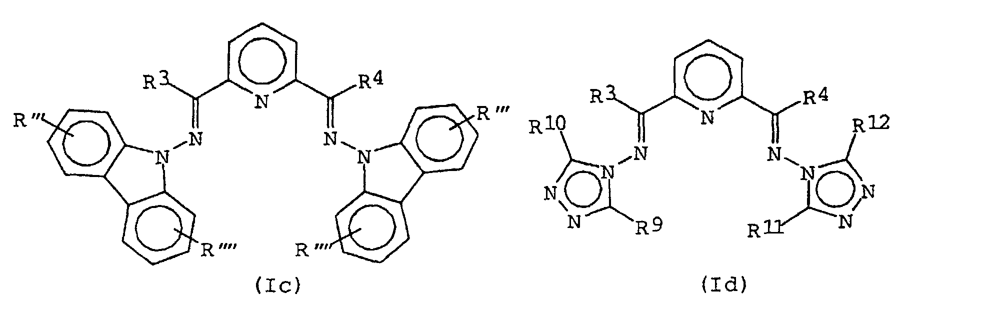 worin
R³, R⁴ unabhängig voneinander H, Alkyl- oder Arylreste,
und
R⁹, R¹⁰, R¹¹ und R¹² unabhängig voneinander C₁- bis C₆-Alkylreste bedeuten, und
R', R'', R''', R'''' H, Alkyl-, Aryl- oder Cycloalkylreste
bedeuten, angehören.

7. Verfahren zur Herstellung von symmetrischen Verbindungen der allgemeinen Formel (I) gemäß Anspruch 1, worin R¹ = R² ist, durch Umsetzung von Verbindungen der allgemeinen Formel (II)
H₂N―NR⁵R⁶ (II)
worin
R⁵ und R⁶ gemeinsam mit dem N-Atom einen 5-, 6- oder 7-gliedrigen Ring bilden, in dem eine oder mehrere der -CH- oder -CH₂-Gruppen durch geeignete Heteroatomgruppen ersetzt sein können, der gesättigt, ungesättigt und unsubstituiert, substituiert oder mit weiteren carbacyclischen oder heterocarbacyclischen 5- oder 6-gliedrigen Ringen, die ihrerseits gesättigt oder ungesättigt und substituiert oder unsubstituiert sein können, anelliert sein kann,
mit Verbindungen der allgemeinen Formel (III) 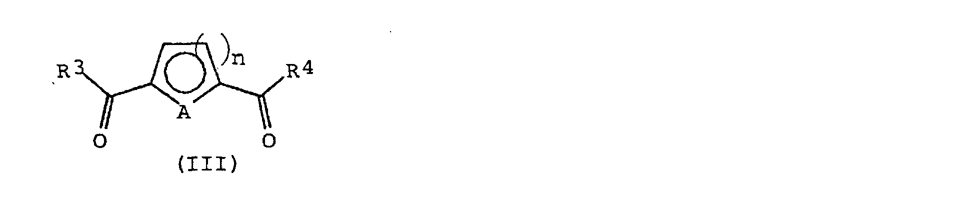 worin
R³, R⁴ unabhängig voneinander H, Alkyl-, Aryl- oder Cycloaklylreste sind, und
A S, N, O oder P ist, und
n 1 oder 2 ist,
in einem einstufigen Verfahren unter sauren Reaktionsbedingungen in alkoholischer Lösung oder unter Aluminiumtrialkyl-Katalyse in einem aprotischen Lösungsmittel in einem Verhältnis der Verbindung der allgemeinen Formel (II) zu der Verbindung der allgemeinen Formel (III) von 2 : 0,7 bis 1,3.

8. Verfahren zur Herstellung von unsymmetrischen Verbindungen der allgemeinen Formel (I) gemäß Anspruch 1, worin R¹ ≠ R² ist, in einem zweistufigen Verfahren, in dem
a) in einer ersten Stufe Verbindungen der allgemeinen Formel (II)
H₂N―NR⁵R⁶ (II)
worin
R⁵ und R⁶ gemeinsam mit dem N-Atom einen 5-, 6- oder 7-gliedrigen Ring bilden, in dem eine oder mehrere der -CH- oder -CH₂-Gruppen durch geeignete Heteroatomgruppen ersetzt sein können, der gesättigt, ungesättigt und unsubstituiert, substituiert oder mit weiteren carbacyclischen oder heterocarbacyclischen 5- oder 6-gliedrigen Ringen, die ihrerseits gesättigt oder ungesättigt und substituiert oder unsubstituiert sein können, anelliert sein kann,
mit Verbindungen der allgemeinen Formel (III) 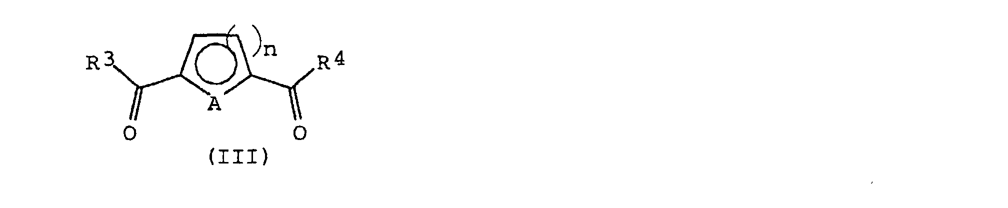 worin
R³, R⁴ unabhängig voneinander H, Alkyl-, Aryl- oder Cycloaklylreste sind, und
A S, N, O oder P ist, und
n 1 oder 2 ist,
in einem Verhältnis der Verbindungen der allgemeinen Formel (II) zu den Verbindungen der allgemeinen Formel (III) von 1 : 0,8 bis 1,2 unter sauren Bedingungen in alkoholischer Lösung zu dem entsprechenden Monoimin umgesetzt werden und das Lösungsmittel anschließend im Vakuum entfernt wird,
und
b) das Monoimin in einer zweiten Stufe mit Verbindungen der allgemeinen Formel (II), die sich von den in Stufe a) eingesetzten Verbindungen der allgemeinen Formel (II) unterscheiden, oder mit Verbindungen der allgemeinen Formel (IV)
H₂N―NR⁷R⁸ (IV)
worin
R⁷ und R⁸ unabhängig voneinander Alkyl-, ein Aryl- oder Cycloalkylreste sind,
oder mit Aminen der allgemeinen Formel (V)
R¹³―NH₂ (V)
worin
R¹³ ein Alkyl-, ein Aryl- oder ein Cycloalkylrest ist,
in aprotischer Lösung unter Aluminiumtrialkylkatalyse in einem Verhältnis des Monoimins zu einer Verbindung der allgemeinen Formel (II), (IV) oder (V) von 1 : 0,8 bis 1,2 umgesetzt wird.

9. Verbindungen der allgemeinen Formel (VI), 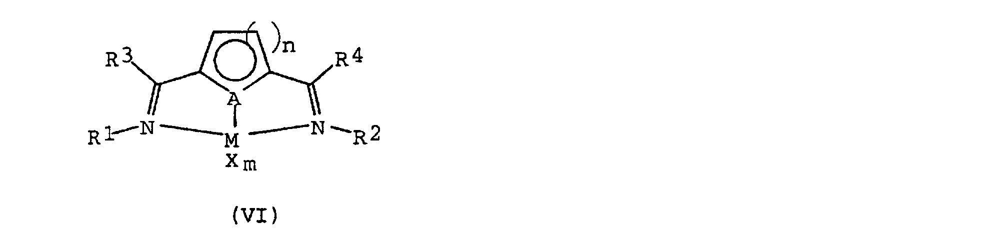 worin die Symbole die folgende Bedeutung haben
A Nichtmetall ausgewählt aus N, S, O und P
R¹ Reste der allgemeinen Formel NR⁵R⁶,
R² Reste der allgemeinen Formel NR⁵R⁶, NR⁷R⁸ oder Alkyl-, Aryl- oder Cycloalkylreste,
R⁵ und R⁶ bilden gemeinsam mit dem N-Atom einen 5-, 6- oder 7-gliedrigen Ring, in dem eine oder mehrere der -CH- oder -CH₂-Gruppen durch geeignete Heteroatomgruppen ersetzt sein können, der gesättigt, ungesättigt und unsubstituiert, substituiert oder mit weiteren carbacyclischen oder heterocarbacyclischen 5- oder 6-gliedrigen Ringen, die ihrerseits gesättigt oder ungesättigt und substituiert oder unsubstituiert sein können, anelliert sein kann, und
R⁷, R⁸ unabhängig voneinander Alkyl-, Aryl- oder Cycloalkylreste,
und
R³, R⁴ unabhängig voneinander H, Alkyl-, Aryl- oder Cycloalkkylreste,
n 1 oder 2;
M Übergangsmetall der Gruppe 7, 8, 9 oder 10 des Periodensystems der Elemente,
und
X ein Halogenid oder ein C₁- bis C₆-Alkylrest,
m Wertigkeit des Metalls.

10. Verbindungen nach Anspruch 9, **dadurch gekennzeichnet, daß** M = Fe oder Co und m = 2 ist.

11. Verfahren zur Herstellung von Verbindungen der allgemeinen Formel (VI) gemäß Anspruch 9, durch Umsetzung von entsprechenden Verbindungen der allgemeinen Formel (I) nach einem der Ansprüche 1 bis 5 mit Übergangsmetallsalzen von Metallen der Gruppen 7, 8, 9 oder 10 des Periodensystems der Elemente.

12. Verwendung von Verbindungen der allgemeinen Formel (VI) gemäß Anspruch 9 oder 10 als Katalysatoren in einem Verfahren zur Polymerisation ungesättigter Verbindungen.

13. Verfahren zur Herstellung von Polyolefinen durch Polymerisation ungesättigter Verbindungen in Anwesenheit eines Aktivators und einer Verbindung der allgemeinen Formel (VI) gemäß Anspruch 9 oder 10 als Katalysator.

14. Verfahren nach Anspruch 13, **dadurch gekennzeichnet, daß** der Katalysator in der Polymerisation homogen in Lösung oder heterogen auf einen Träger immobilisiert vorliegt.

15. Verfahren nach Anspruch 13 oder 14, **dadurch gekennzeichnet, daß** Methylaluminoxan oder N,N-Dimethylanilinium-tetrakis(pentafluorophenyl)borat als Aktivator eingesetzt wird.

16. Verfahren nach einem der Ansprüche 13 bis 15, **dadurch gekennzeichnet, daß** eine ungesättigte Verbindung oder eine Kombination ungesättigter Verbindungen ausgewählt aus Ethylen, C₃bis C₂₀-Monoolefinen, und Cycloolefinen eingesetzt wird.

17. Verfahren nach einem der Ansprüche 13 bis 15, **dadurch gekennzeichnet, daß** Acrylnitril und Stryrol als Comonomere eingesetzt werden oder folgende Kombinationen ungesättigter Verbindungen: Ethylen und ein Alkylacrylat, insbesondere Methylacrylat, Ethylen und eine Acrylsäure, Ethylen und Kohlenmonoxid, Ethylen, Kohlenmonoxid und ein Acrylatester oder eine Acrylsäure, insbesondere Methylacrylat sowie Propylen und Alkylacrylat, insbesondere Methylacrylat.

18. Polyolefin, herstellbar in einem Verfahren nach einem der Ansprüche 13bis 17.

## Claims

1. A compound of the formula (I) 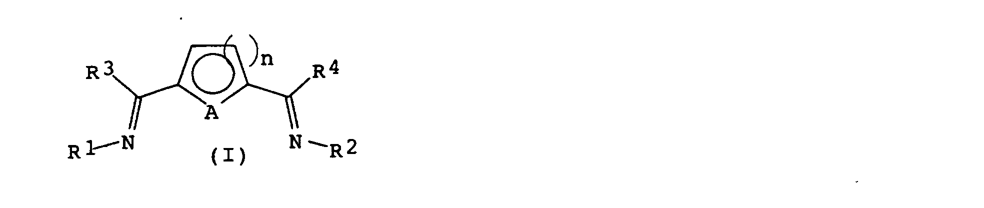 where the symbols have the following meanings:
A is a nonmetal selected from among N, S, O and P,
R¹ is a radical of the formula NR⁵R⁶,
R² is a radical of the formula NR⁵R⁶ or NR⁷R⁸, alkyl, aryl or cycloalkyl,
R⁵ and R⁶ together with the N atom form a 5-, 6- or 7-membered ring in which one or more of the -CH- or -CH₂- groups may be replaced by suitable heteroatom groups and which may be saturated or unsaturated and unsubstituted or substituted or be fused with further carbacyclic or heterocarbacyclic 5- or 6-membered rings which may in turn be saturated or unsaturated and substituted or unsubstituted, and
R⁷ and R⁸ are, independently of one another, alkyl, aryl or cycloalkyl radicals,
and
R³, R⁴ are, independently of one another, H or alkyl, aryl or cycloalkyl radicals,
and
n is 1 or 2.

2. A compound as claimed in claim 1, wherein the radicals of the formula NR⁵R⁶ are pyrrole radicals or radicals derived from pyrrole in which one or more -CH- groups in the pyrrole ring may be replaced by nitrogen and which may be unsubstituted or substituted or fused with further carbacyclic or heterocarbacyclic 5- or 6-membered rings which may in turn be saturated or unsaturated and substituted or unsubstituted.

3. A compound as claimed in claim 2, wherein the pyrrole radicals or radicals derived from pyrrole are substituted in the 2 and 5 positions by C₁-C₆-alkyl groups which may be linear, branched or substituted by heteroatoms, and/or by aryl groups which may be unsubstituted or in turn substituted by heteroatoms or C₁-C₆-alkyl groups which may be heteroatom-substituted.

4. A compound as claimed in claim 1 or 2, wherein the pyrrole radicals or radicals derived from pyrrole are substituted in the 2 or 5 position by electron-withdrawing radicals selected from among
- halogen,
- NO₂,
- sulfonates selected from among
- SO₃R*,
- SO₃SiR*₃ and
- SO₃⁻ (H-NR*₃)⁺,
- -trihalomethyl,
where R* may be identical or different and are selected from among H, C₁-C₁₀-alkyl, C₆-C₂₀-aryl and C₅-C₈-cycloalkyl.

5. A compound as claimed in any of claims 1 to 4, wherein, in the formula (I) of claim 1, A = N and n = 2.

6. A compound as claimed in claim 5 which corresponds to one of the formulae (Ia), (Ib), (Ic) and (Id): 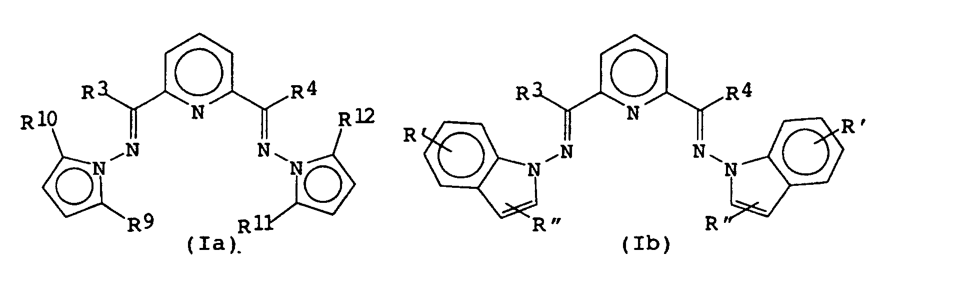 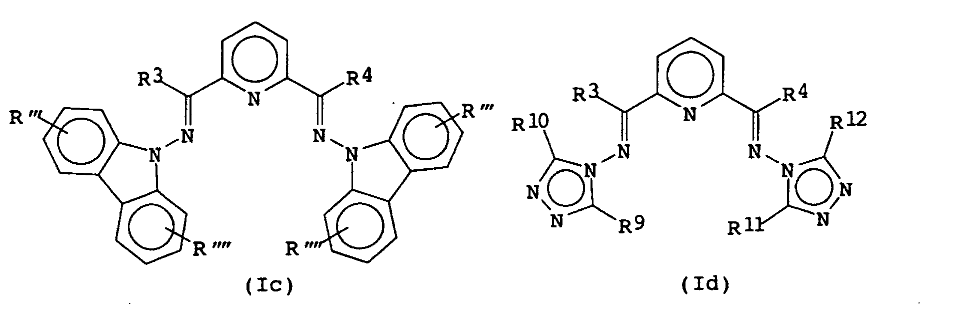 where
R³, R⁴ are, independently of one another, H or alkyl or aryl radicals,
and
R⁹, R¹⁰, R¹¹ and R¹² are, independently of one another, C₁-C₆-alkyl radicals, and
R', R'', R''', R'''' are H or alkyl, aryl or cycloalkyl radicals.

7. A process for preparing symmetrical compounds of the formula (I) of claim 1 in which R¹ = R² by reacting compounds of the formula (II)
H₂N―NR⁵R⁶ (II)
where
R⁵ and R⁶ together with the N atom form a 5-, 6- or 7-membered ring in which one or more of the -CH- or -CH₂- groups may be replaced by suitable heteroatom groups and which may be saturated or unsaturated and unsubstituted or substituted or fused with further carbacyclic or heterocarbacyclic 5- or 6-membered rings which may in turn be saturated or unsaturated and substituted or unsubstituted,
with compounds of the formula (III) 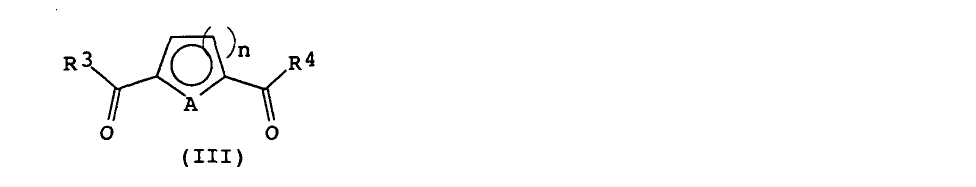 where
R³, R⁴ are, independently of one another, H or alkyl, aryl or cycloaklyl radicals, and
A is S, N, O or P, and,
n is 1 or 2, and
in a single-stage process under acidic reaction conditions in alcoholic solution or in the presence of a trialkylaluminum catalyst in an aprotic solvent in a ratio of the compound of the formula (II) to the compound of the formula (III) of 2:0.7-1.3.

8. A process for preparing unsymmetrical compounds of the formula (I) of claim 1 in which R¹ ≠ R² in a two-stage process in which
a) in a first step, compounds of the formula (II)
H₂N―NR⁵R⁶ (II)
where
R⁵ and R⁶ together with the N atom form a 5-, 6- or 7-membered ring in which one or more of the -CH- or -CH₂- groups may be replaced by suitable heteroatom groups and which may be saturated or unsaturated and substituted or unsubstituted or fused with further carbacyclic or heterocarbacyclic 5- or 6-membered rings which may in turn be saturated or unsaturated and substituted or unsubstituted,
are reacted with compounds of the formula (III) 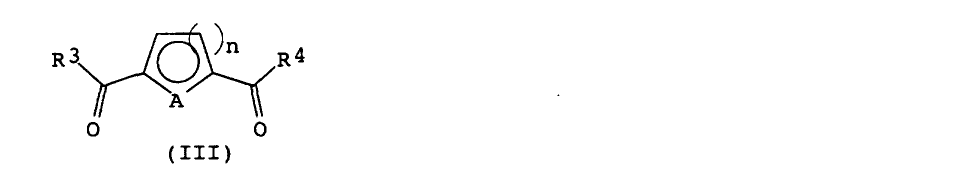 where
R³, R⁴ are, independently of one another, H or alkyl, aryl or cycloalkyl radicals, and
A is S, N, O or P, and
n is 1 or 2,
in a ratio of the compounds of the formula (II) to the compounds of the formula (III) of 1:0.8-1.2 under acidic conditions in alcoholic solution to form the corresponding monoimine and the solvent is subsequently removed under reduced pressure,
and
b) the monoimine is, in a second step, reacted with compounds of the formula (II) which differ from the compounds of the formula (II) used in step a) or with compounds of the formula (IV)
H₂N ― NR⁷R⁸ (IV)
where
R⁷ and R⁸ are, independently of one another, alkyl, aryl or cycloalkyl radicals,
or with amines of the formula (V)
R¹³―NH₂ (V)
where
R¹³ is an alkyl, aryl or cycloalkyl radical,
in aprotic solution in the presence of a trialkylaluminum catalyst in a ratio of the monoimine to the compound of the formula (II), (IV) or (V) of 1:0.8-1.2.

9. A compound of the formula (VI), 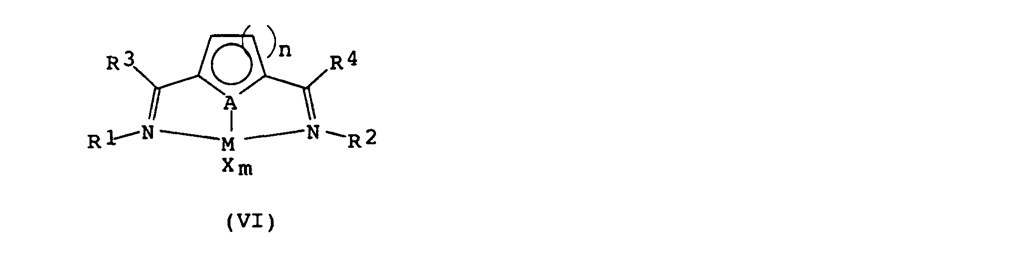 where the symbols have the following meanings:
A is a nonmetal selected from among N, S, O and P,
R¹ is a radical of the formula NR⁵R⁶,
R² is a radical of the formula NR⁵R⁶ or NR⁷R⁸, alkyl, aryl or cycloalkyl,
R⁵ and R⁶ together with the N atom form a 5-, 6- or 7-membered ring in which one or more of the -CH- or -CH₂- groups may be replaced by suitable heteroatom groups and which may be saturated or unsaturated and unsubstituted or substituted or be fused with further carbacyclic or heterocarbacyclic 5- or 6-membered rings which may in turn be saturated or unsaturated and substituted or unsubstituted, and
R⁷ and R⁸ are, independently of one another, alkyl, aryl or cycloalkyl radicals,
and
R³, R⁴ are, independently of one another, H or alkyl, aryl or cycloalkyl radicals,
n is 1 or 2,
M is a transition metal of groups 7, 8, 9 or 10 of the Periodic Table of the Elements,
and
X is a halide or a C₁-C₆-alkyl radical and
m is the valence of the metal.

10. A compound as claimed in claim 9, wherein
M = Fe or Co and m = 2.

11. A process for preparing compounds of the formula (VI) of claim 9 by reacting corresponding compounds of the formula (I) as claimed in any of claims 1 to 5 with salts of transition metals of groups 7, 8, 9 or 10 of the Periodic Table of the Elements.

12. The use of compounds of the formula (VI) as claimed in claim 9 or 10 as catalysts in a process for the polymerization of unsaturated compounds.

13. A process for preparing polyolefins by polymerization of unsaturated compounds in the presence of an activator and a compound of the formula (VI) as claimed in claim 9 or 10 as catalyst.

14. A process as claimed in claim 13, wherein the catalyst is present in the polymerization either as a homogeneous solution or in heterogeneous form immobilized on a support.

15. A process as claimed in claim 13 or 14, wherein methylaluminoxane or N,N-dimethylanilinium tetrakis(pentafluorophenyl)borate is used as activator.

16. A process as claimed in any of claims 13 to 15, wherein an unsaturated compound or a combination of unsaturated compounds selected from among ethylene, C₃-C₂₀-monoolefins and cycloolefins is used.

17. A process as claimed in any of claims 13 to 15, wherein acrylonitrile and styrene are used as comonomers or the following combinations of unsaturated compounds are employed: ethylene and an alkyl acrylate, in particular methyl acrylate, ethylene and an acrylic acid, ethylene and carbon monoxide, ethylene, carbon monoxide and an acrylate ester or an acrylic acid, in particular methyl acrylate, and also propylene and alkyl acrylate, in particular methyl acrylate.

18. A polyolefin which can be prepared in a process as claimed in any of claims 13 to 17.

## Revendications

1. Composés de la formule générale (I) : 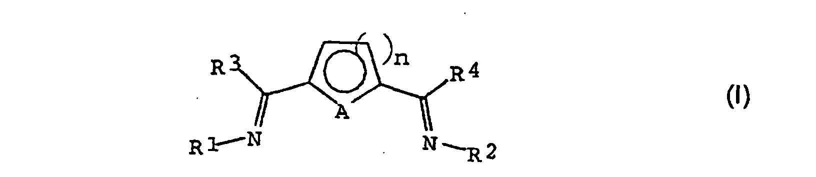 dans laquelle les symboles ont la signification suivante :
A un métalloïde choisi parmi N, S, O et P,
R¹ des radicaux de la formule générale NR⁵R⁶,
R² des radicaux de la formule générale NR⁵R⁶ ou NR⁷R⁸, ou des radicaux alkyle, aryle ou cycloalkyle,
R⁵ et R⁶ forment conjointement avec l'atome de N un noyau pentagonal, hexagonal ou heptagonal, dans lequel un ou plusieurs des groupes -CH- ou -CH₂- peuvent être remplacés par des groupes hétéro-atomiques appropriés, noyau qui peut être saturé, insaturé et non substitué, substitué ou annelé avec d'autres noyaux carbacycliques ou hétérocarbacyliques pentagonaux ou hexagonaux qui, à leur tour, peuvent être saturés ou insaturés et substitués ou non substitués, et
R⁷ et R⁸, indépendamment l'un de l'autre, des radicaux alkyle, aryle ou cycloalkyle, et
R³ et R⁴, indépendamment l'un de l'autre, H, ou des radicaux alkyle, aryle ou cycloalkyle, et
n 1 ou 2.

2. Composés suivant la revendication 1, **caractérisés en ce que** les radicaux de la formule générale NR⁵R⁶ sont des radicaux de pyrrole ou dérivés de pyrrole, dans lesquels un ou plusieurs groupes -CH- peuvent être échangés dans le noyau de pyrrole par de l'azote, radicaux qui peuvent être non substitués, substitués ou annelés avec d'autres noyaux carbacycliques ou hétérocarbacyliques pentagonaux ou hexagonaux, qui, à leur tour, peuvent être saturés ou non saturés et substitués ou non substitués.

3. Composés suivant la revendication 2, **caractérisés en ce que** les radicaux de pyrrole ou dérivés de pyrrole sont, dans les positions 2 et 5, substitués avec des groupes alkyle en C₁-C₆, qui peuvent être linéaires, ramifiés et substitués par des hétéroatomes, et/ou avec des groupes aryle qui sont non substitués ou, à leur tour, substitués par des hétéroatomes ou des groupes alkyle en C₁-C₆, qui peuvent être substitués par des hétéroatomes.

4. Composés suivant l'une des revendications 1 et 2, **caractérisés en ce que** les radicaux de pyrrole ou les radicaux dérivés de pyrrole sont substitués dans la position 2 ou 5 par des radicaux attirant les électrons, choisis parmi
- de l'halogène,
- NO₂,
- des sulfonates, choisis parmi
- SO₃R*,
- SO₃SiR*₃ OU
- SO₃⁻(H-NR*₃)⁺
- du trihalogénométhyle,
où R* peuvent être identiques ou différents et choisis parmi H, de l'alkyle en C₁-C₁₀, de l'aryle en C₆-C₂₀ ou du cycloalkyle en C₅-C₈.

5. Composés suivant l'une des revendications 1 à 4, **caractérisés en ce que**, dans la formule générale (I) suivant la revendication 1, A = N et n = 2.

6. Composés suivant la revendication 5, **caractérisés en ce qu'**ils appartiennent à une des formules générales (Ia), (Ib), (Ic) ou (Id) : 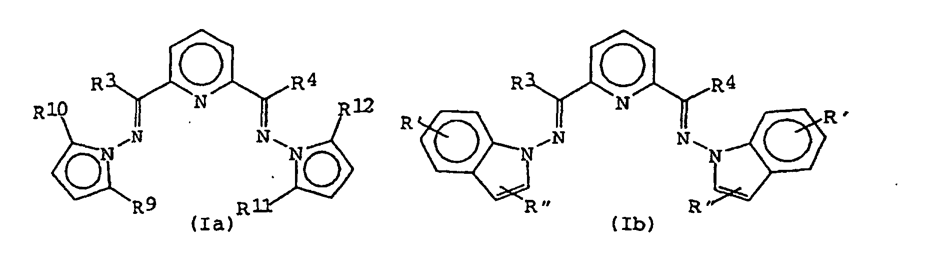 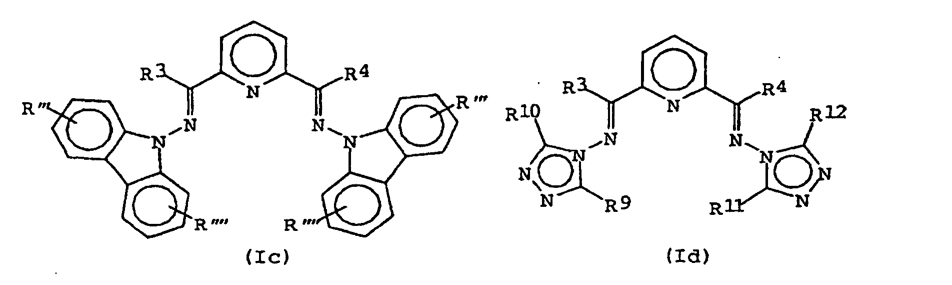 dans lesquelles
R³, R⁴ représentent, indépendamment l'un de l'autre, H, ou des radicaux alkyle ou aryle, et
R⁹, R¹⁰, R¹¹ et R¹² représentent, indépendamment l'un de l'autre, des radicaux alkyle en C₁-C₆, et
R', R", R"', R"" représentent H, ou des radicaux alkyle, aryle ou cycloalkyle.

7. Procédé de préparation de composés symétriques de la formule générale (I) suivant la revendication 1, dans laquelle R¹ = R², par réaction de composés de la formule générale (II) :
H₂N ― NR⁵R⁶ (II)
dans laquelle
R⁵ et R⁶ forment conjointement avec l'atome de N un noyau pentagonal, hexagonal ou heptagonal, dans lequel un ou plusieurs groupes -CH- ou -CH₂- peuvent être remplacés par des groupes hétéroatomiques appropriés, noyau qui peut être saturé, insaturé et non substitué, substitué ou annelé avec d'autres noyaux pentagonaux ou hexagonaux carbacycliques ou hétérocarbacyliques, qui, à leur tour, peuvent être saturés ou insaturés et substitués ou non substitués,
avec des composés de la formule générale (III) : 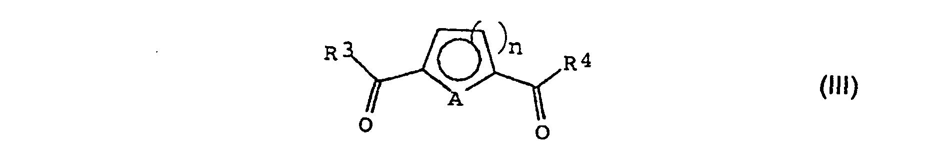 dans laquelle
R³, R⁴ représentent, indépendamment l'un de l'autre, H, ou des radicaux alkyle, aryle ou cycloalkyfe, et
A représente S, N, O ou P, et
n est égal à 9 ou 2,
dans un procédé en une étape dans des conditions réactionnelles acides en solution alcoolique ou sous catalyse par de l'aluminium-trialkyle dans un solvant aprotique, dans un rapport entre le composé de la formule générale (II) et le composé de la formule générale (III) de 2/0,7 à 1,3.

8. Procédé de préparation de composés asymétriques de la formule générale (I) suivant la revendication 1, dans laquelle R¹≠R², dans un procédé en deux étapes, dans lequel
a) dans une première étape, on fait réagir dans des conditions acides en solution alcoolique, pour former la monoimine correspondante, des composés de la formule générale (II) :
H₂N―NR⁵R⁶ (II)
dans laquelle
R⁵ et R⁶ forment conjointement avec l'atome de N un noyau pentagonal, hexagonal ou heptagonal, dans lequel un ou plusieurs groupes -CH- ou -CH₂- peuvent être remplacés par des groupes hétéroatomiques appropriés, noyau qui peut être saturé, insaturé et non substitué, substitué ou annelé avec d'autres noyaux pentagonaux ou hexagonaux carbacycliques ou hétérocarbacyliques, qui, à leur tour, peuvent être saturés ou insaturés et substitués ou non substitués,
avec des composés de la formule générale (III): 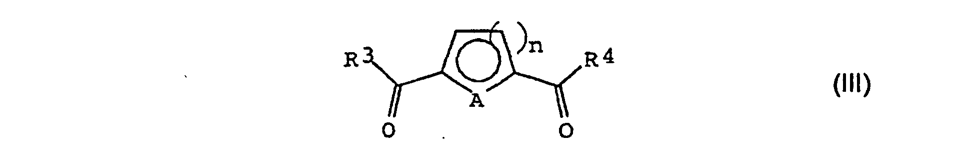 dans laquelle
R³, R⁴ représentent, indépendamment l'un de l'autre, H, ou des radicaux alkyle, aryle ou cycloalkyle, et
A représente S, N, O ou P, et
n est égal à 1 ou 2,
dans un rapport entre les composés de la formule générale (II) et les composés de la formule générale (III) de 1/0,8 à 1,2, et on élimine ensuite le solvant sous vide,
et
b) dans une deuxième étape, en solution aprotique, sous catalyse par de l'aluminium-trialkyle, on fait réagir la monoimine avec des composés de la formule générale (II), qui se différencient des composés de la formule générale (II) mis en oeuvre dans l'étape a), ou avec des composés de la formule générale (IV):
H₂N ― NR⁷R⁸ (IV)
dans laquelle
R⁷ et R⁸ représentent, indépendamment l'un de l'autre, des radicaux alkyle, aryle ou cycloalkyle,
ou avec des amines de la formule générale (V) :
R¹³―NH₂ (V)
dans laquelle
R¹³ représente un radical alkyle, aryle ou cycloalkyle, dans un rapport entre la monoimine et un composé de la formule générale (II), (IV) ou (V) de 1/0,8 à 1,2.

9. Composés de la formule générale (VI) : 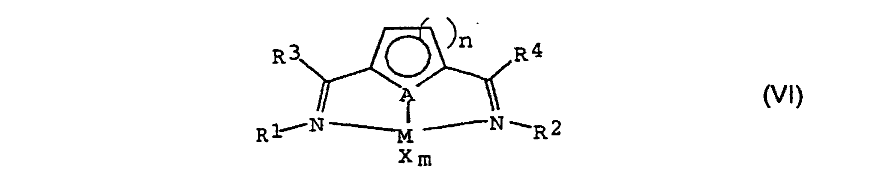 dans laquelle les symboles ont la signification suivante :
A un métalloïde choisi parmi N, S, O et P,
R¹ des radicaux de la formule générale NR⁵R⁶,
R² des radicaux de la formule générale NR⁵R⁶, NR⁷R⁸ ou des radicaux alkyle, aryle ou cycloalkyle,
R⁵ et R⁶ forment conjointement avec l'atome de N un noyau pentagonal, hexagonal ou heptagonal, dans lequel un ou plusieurs des groupes -CH- ou -CH₂- peuvent être remplacés par des groupes hétéro-atomiques appropriés, noyau qui peut être saturé, insaturé et non substitué, substitué ou annelé avec d'autres noyaux pentagonaux ou hexagonaux carbacycliques ou hétérocarbacyliques, qui, à leur tour, peuvent être saturés ou insaturés et substitués ou non substitués, et
R⁷, R⁸ représentent, indépendamment l'un de l'autre, des radicaux alkyle, aryle ou cycloalkyle, et
R³ et R⁴ représentent, indépendamment l'un de l'autre, H, ou des radicaux alkyle, aryle ou cycloalkyle,
n 1 ou 2,
M un métal de transition des groupes 7, 8, 9 ou 10 du Système Périodique des Eléments,
et
X un halogénure ou un radical alkyle en C₁-C₆,
m une valence du métal.

10. Composés suivant la revendication 9, **caractérisés en ce que** M = Fe ou Co et m = 2.

11. Procédé de préparation de composés de la formule générale (VI) suivant la revendication 9, par réaction de composés correspondants de la formule générale (I) suivant une des revendications 1 à 5, avec des sels de métaux de transition des groupes 7, 8, 9 ou 10 du Système Périodique des Eléments.

12. Utilisation des composés de la formule générale (VI) suivant l'une des revendications 9 et 10, comme catalyseurs dans un procédé de polymérisation de composés insaturés.

13. Procédé de préparation de polyoléfines par polymérisation de composés insaturés, en présence d'un activeur et d'un composé de la formule générale (VI) suivant l'une des revendications 9 et 10, comme catalyseur.

14. Procédé suivant la revendication 13, **caractérisé en ce que** le catalyseur se trouve dans la polymérisation d'une manière homogène en solution ou immobilisé de manière hétérogène sur un support.

15. Procédé suivant l'une des revendications 13 et 14, **caractérisé en ce que**, comme activeur, on met en oeuvre du méthylaluminoxane ou du borate de N,N-diméthylanilinium-tétra-(pentafluorophényle).

16. Procédé suivant l'une des revendications 13 à 15, **caractérisé en ce qu'**on met en oeuvre un composé insaturé ou une combinaison de composés insaturés choisis parmi de l'éthylène, des monooléfines en C₃-C₂₀ et des cyclooléfines.

17. Procédé suivant l'une des revendications 13 à 15, **caractérisé en ce qu'**on met en oeuvre de l'acrylonitrile et du styrène, comme comonomères, ou les combinaisons suivantes de composés insaturés : de l'éthylène et un acrylate d'alkyle, en particulier de l'acrylate de méthyle, de l'éthylène et un acide acrylique, de l'éthylène et du monoxyde de carbone, de l'éthylène, du monoxyde de carbone et un acrylate ou un acide acrylique, en particulier de l'acrylate de méthyle, ainsi que du propylène et un acrylate d'alkyle, en particulier de l'acrylate de méthyle.

18. Polyoléfine, que l'on peut préparer suivant un procédé selon l'une des revendications 13 à 17.
